(19) Europäisches Patentamt
European Patent Office
Office européen des brevets

(11) EP 0 983 992 B1

(12) EUROPEAN PATENT SPECIFICATION

(45) Date of publication and mention of the grant of the patent:
**26.10.2005 Bulletin 2005/43**

(21) Application number: **99118828.5**

(22) Date of filing: **22.04.1993**

(51) Int Cl.[7]: **C07C 63/49**, A61K 31/19, A61K 31/44, C07C 63/72, C07C 65/40, C07C 69/76, C07C 235/84, C07D 307/68, C07D 333/38, C07D 213/80, C07D 311/58, C07D 213/79, C07D 207/40

(54) **Compounds having selectivity for retinoid x receptors**

Verbindungen mit Retinoid x rezeptorselektivität

Composés à sélectivité pour les récepteurs rétinoide x

(84) Designated Contracting States:
**AT BE CH DE DK ES FR GB GR IE IT LI LU MC NL PT SE**

(30) Priority:
**22.04.1992 US 872707**
**11.09.1992 US 944783**
**11.01.1993 US 3223**
**05.03.1993 US 27747**
**21.04.1993 US 52051**

(43) Date of publication of application:
**08.03.2000 Bulletin 2000/10**

(62) Document number(s) of the earlier application(s) in accordance with Art. 76 EPC:
**93910835.3 / 0 637 297**

(73) Proprietor: **LIGAND PHARMACEUTICALS INCORPORATED**
**San Diego, California 92121 (US)**

(72) Inventors:
- **Boehm, Marcus F.**
  **San Diego, CA 92129 (US)**
- **Heyman, Richard A.**
  **Encinitas, CA 92024 (US)**
- **Zhi, Lin**
  **San Diego, CA 92129 (US)**

(74) Representative: **Keen, Celia Mary et al**
**J.A. Kemp & Co.**
**14 South Square**
**Gray's Inn**
**London WC1R 5JJ (GB)**

(56) References cited:
**EP-A- 0 260 162**
**WO-A-94/12880**
**DE-A- 3 711 546**
**FR-A- 2 307 524**
**US-A- 4 548 723**
**WO-A-00/20358**
**WO-A-98/46076**
**FR-A- 1 444 545**
**GB-A- 2 197 316**

- **DAWSON, MARCIA I. ET AL: "Conformational Effects on Retinoid Receptor Selectivity. 2. Effects of Retinoid Bridging Group on Retinoid X Receptor Activity and Selectivity" J. MED. CHEM., vol. 38, no. 17, 1995, pages 3368-3383, XP002148373**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 983 992 B1

Printed by Jouve, 75001 PARIS (FR)

## Description

This application is a continuation-in-part of the application Serial No. 08/027,747, filed on March 5, 1993, which is a continuation-in-Part of application Serial No. 08/003,223, filed on January 11, 1993, which is a continuation-in-part of application Serial No. 944,783, filed on September 11, 1992, which is a continuation-in-part of application Serial No. 872,707 filed April 22, 1992.

This invention relates to intracellular receptors and ligands therefor. More specifically, this invention relates to compounds having selective activity for specific retinoic acid receptors, and methods for use of such compounds.

The vitamin A metabolite retinoic acid has long.been recognized to induce a broad spectrum of biological effects. A variety of structural analogues of retinoic acid have been synthesized that also have been found to be bioactive. Some, such as Retin-A® (registered trademark of Johnson & Johnson) and Accutane® (registered trademark of Hoffmann-LaRoche), have found utility as therapeutic agents for the treatment of various pathological conditions. Metabolites of vitamin A and their synthetic analogues are collectively herein called "retinoids".

Synthetic retinoids have been found to mimic many of the pharmacological actions of retinoic acid. However, the broad spectrum of pharmacological actions of retinoic acid is not reproduced in full by all bioactive synthetic retinoids.

Medical professionals have become very interested in the medicinal applications of retinoids. Among their uses approved by the FDA is the treatment of severe forms of acne and psoriasis. A large body of evidence also exists that these compounds can be used to arrest and, to an extent, reverse the effects of skin damage arising from prolonged exposure to the sun. Other evidence exists that these compounds may be useful in the treatments of a variety of severe cancers including melanoma, cervical cancer, some forms of leukemia, and basal and squamous cell carcinomas. Retinoids have also shown an ability to be efficacious in treating premalignant cell lesions, such as oral leukoplakia, and to prevent the occurrence of malignancy.

Use of the retinoids is associated with a number of significant side effects. The most serious of these is that, as a class, they are among the most potent teratogens known. Teratogens are compounds that cause severe birth defects during specific periods of fetal exposure. Other side effects include irritation of the tissues treated, which can be so severe that patients cannot tolerate treatment.

Various investigations have been undertaken to elucidate the structure-activity relationships governing the abilities of synthetic retinoids to induce the various pharmacological consequences of retinoic acid exposure. This has been a complicated task, however, since the assays available to investigators have been bioassays, carried out either in intact animals or in isolated tissues. Technical constraints have often dictated the use of different small animal species for different assays. Interpretation of results has been complicated by possible pharmacokinetic and metabolic effects and possible species differences in the receptors involved. Nevertheless, definite differences in the pharmacological effects of various synthetic retinoids have been observed.

Major insight into the molecular mechanism of retinoic acid signal transduction was gained in 1988. Prior to that time, several high abundance cellular retinoid binding proteins were incorrectly inferred to be the signal transducing receptors for retinoic acid. In 1988, a member of the steroid/thyroid hormone intra-cellular receptor superfamily (Evans, *Science*, 240:889-95 (1988)) was shown to transduce a retinoic acid signal (Giguere et al., *Nature*, 330:624-29 (1987); Petkovich *et al*., *Nature*, 330: 444-50 (1987)). This unexpected finding related retinoic acid to other non-peptide hormones and elucidated the mechanism of retinoic acid effects in altering cell function. It is now known that retinoids regulate the activity of two distinct intracellular receptor subfamilies; the Retinoic Acid Receptors (RARs) and the Retinoid X Receptors (RXRs).

The first retinoic acid receptor identified, designated RAR-alpha, acts to modulate transcription of specific target genes in a manner which is ligand-dependent, as has been shown to be the case for many of the members of the steroid/thyroid hormone intracellular receptor superfamily. The endogenous low-molecular-weight ligand upon which the transcription-modulating activity of RAR-alpha depends is all-*trans*-retinoic acid. Retinoic acid receptor-mediated changes in gene expression result in characteristic alterations in cellular phenotype, with consequences in many tissues manifesting the biological response to retinoic acid. Two additional genes closely related to RAR-alpha were recently identified and were designated RAR-beta and RAR-gamma and are very highly related (Brand *et al*., *Nature*, 332:850-53 (1988); Ishikawa *et al*., *Mol. Endocrin*., 4:837-44 (1990)). In the region of the retinoid receptors which can be shown to confer ligand binding, the primary amino acid sequences diverge by less than 15% among the three RAR subtypes or isoforms. All-*trans*-retinoic acid is a natural ligand for the retinoic acid receptors (RARs) and is capable of binding to these receptors with high affinity, resulting in the regulation of gene expression. The newly-discovered retinoid metabolite, 9-*cis*-retinoic acid, is also an activator of RARs.

A related but unexpected observation was made recently (Mangelsdorf *et al., Nature,* 345 :224-29 (1990)), in which another member of the steroid/thyroid receptor superfamily was also shown to be responsive to retinoic acid. This new retinoid receptor subtype has been designated Retinoid X Receptor (RXR), because certain earlier data suggested that a derivative of all-*trans*-retinoic acid may be the endogenous ligand for RXR. Like the RARs, the RXRs

are also known to have at least three subtypes or isoforms, namely RXR-alpha, RXR-beta, and RXR-gamma, with corres-ponding unique patterns of expression (Manglesdorf et al., *Genes & Devel.*, 6:329-44 (1992)).

Although both the RARs and RXRs respond to all-*trans*-retinoic acid *in vivo*, the receptors differ in several important aspects. First, the RARs and RXRs are significantly divergent in primary structure (*e.g.*, the ligand binding domains of RARα and RXRα have only 27% amino acid identity). These structural differences are reflected in the different relative degrees of responsiveness of RARs and RXRs to various vitamin A metabolites and synthetic retinoids. In addition, distinctly different patterns of tissue distribution are seen for RARs and RXRs. For example, in contrast to the RARs, which are not expressed at high levels in the visceral tissues, RXRα mRNA has been shown to be most abundant in the liver, kidney, lung, muscle and intestine. Finally, the RARs and RXRs have different target gene specificity. For example, response elements have recently been identified in the cellular retinal binding protein type II (CRBPII) and apolipoprotein AI genes which confer responsiveness to RXR, but not RAR. Furthermore, RAR has also been recently shown to repress RXR-mediated activation through the CRBPII RXR response element (Manglesdorf *et al*., *Cell*, 66:555-61 (1991)). These data indicate that two retinoic acid responsive pathways are not simply redundant, but instead manifest a complex interplay. Recently, Heyman *et al*. (*Cell*, 68:397-406 (1992)) and Levin *et al*. (*Nature*, 355:359-61 (1992)) independently demonstrated that 9-*cis*-retinoic acid is a natural endogenous ligand for the RXRs. 9-*cis*-retinoic acid was shown to bind and transactivate the RXRs, as well as the RARs, and therefore appears to act as a "bifunctional" ligand.

In view of the related, but clearly distinct, nature of these receptors, ligands which are more selective for the Retinoid X Receptor subfamily would be of great value for selectively controlling processes mediated by one or more of the RXR isoforms, and would provide the capacity for independent control of the physiologic processes mediated by the RXRs. Ligands which preferentially affect one or more but not all of the receptor isoforms also offer the possibility of increased therapeutic efficacy when used for medicinal applications.

The present invention is directed to compounds, compositions, and methods for modulating processes mediated by one or more Retinoid X Receptors. More particularly, the invention relates to compounds which selectively or preferentially activate Retinoid X Receptors, in comparison to Retinoic Acid Receptors. These compounds selectively modulate processes mediated by Retinoid X Receptors. Accordingly, the invention also relates to methods for modulating processes selectively mediated by one or more Retinoid X Receptors, in comparison to Retinoic Acid Receptors, by use of the compounds of this invention. Examples of compounds used in and forming part of the invention include bicyclic thiophene derivatives. Pharmaceutical compositions containing the compounds disclosed are also within the scope of this invention. Also included are methods for identifying or purifying Retinoid X Receptors by use of the compounds of this invention.

The present invention may be better understood and its advantages appreciated by those skilled in the art by referring to the accompanying drawings wherein:

Figure 1 presents the standardized dose response profiles showing the transactivation of RAR and RXR isoforms by 3-methyl-TTNCB.

Figure 2 presents the standardized dose response profiles showing the transactivation of RAR and RXR isoforms by all-*trans*-retinoic acid.

Figure 3 presents the standardized dose response profiles showing the transactivation of RAR and RXR isoforms by 9-*cis*-retinoic acid.

Figure 4 presents the standardized dose response profiles showing the transactivation of RAR and RXR isoforms by 3-methyl-TTNEB.

Figure 5 presents the standardized dose response profiles showing the transactivation of RAR and RXR isoforms by 3-bromo-TTNEB.

Figure 6 presents the standardized dose response profiles showing the transactivation of RAR and RXR isoforms by 3-methyl-TTNCHBP.

Figure 7 presents the standardized dose response profiles showing the transactivation of RAR and RXR isoforms by 3-methyl-TTNEHBP.

Figure 8 presents the inhibition of transglutaminase activity by 9-*cis*-retinoic acid, all-*trans*-retinoic acid, and 3-methyl-TTNCB.

Figure 9 presents the Topical Dose Response, based on the test on Rhino mice, for 9-*cis*-retinoic acid, all-*trans*-retinoic acid, 3-methyl-TTNCB, 1, 25-dihydroxy Vitamin D.

Figure 10 presents the effect on rat HDL cholesterol of all-*trans*-retinoic acid, 9-*cis*-retinoic acid, 3-methyl-TTNCB, and 3-methyl-TTNEB.

Figure 11 presents the concentration-related effect of 3-methyl-TTNEB and TTNPB individually on incorporation of radiolabeled thymidine into DNA.

Figure 12 presents the concentration-related effect of a combination of 3-methyl-TTNEB and TTNPB on incorporation of radiolabeled thymidine into DNA.

This invention discloses retinoid-like compounds or ligands which have selective activity for members of the subfamily of Retinoid X Receptors (RXRs), in comparison to members of the subfamily of Retinoic Acid Receptors (RARs). These compounds are bicyclic thiophene derivatives. Accordingly, the present invention provides a compound having the formula:

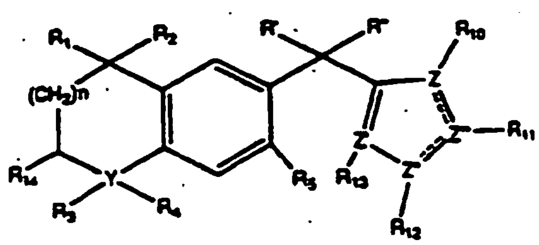

wherein

$R_1$ and $R_2$, each independently, represent hydrogen or lower alkyl or acyl having 1-4 carbon atoms;
Y represents C, O, S, N, CHOH, CO, SO, $SO_2$, or a pharmaceutically acceptable salt;
$R_3$ represents hydrogen or lower alkyl having 1-4 carbon atoms where Y is C or N;
$R_4$ represents hydrogen or lower alkyl having 1-4 carbon atoms where Y is C, but $R_4$ does not exist if Y is N, and neither $R_3$ or $R_4$ exist if Y is S, O, CHOH, CO, SO, or $SO_2$;
R' and R'' represent hydrogen, lower alkyl or acyl having 1-4 carbon atoms, OH, alkoxy having 1-4 carbon atoms, thiol or thio ether, or amino,
or R' and R'' taken together form an oxo (keto), methano, thioketo, HO-N=, NC-N=, $(R_7R_8)$N-N=, epoxy, cyclopropyl, or cycloalkyl group and wherein the epoxy, cyclopropyl, and cycloalkyl groups can be substituted with lower alkyl having 1-4 carbons or halogen;
$R_5$ represents hydrogen, a lower alkyl having 1-4 carbons, halogen, nitro, $OR_7$ $SR_7$, $NR_7R_8$, or $(CF)_nCF_3$;
$R_{10}$, $R_{11}$, $R_{12}$ and $R_{13}$ each independently represent hydrogen, a lower alkyl having 1-4 carbons, halogen, nitro, $OR_7$, $SR_7$, $NR_7R_8$ or $(CF_2)_nCF_3$, if the Z, Z', Z'' or Z''' from which it originates is C, with the proviso that one of $R_{10}$, $R_{11}$, $R_{12}$ and $R_{13}$ is X;
$R_7$ and $R_8$ independently represents hydrogen or a lower alkyl having 1-6 carbons;
$R_{14}$ represents hydrogen, a lower alkyl having 1-4carbons, oxo, hydroxy, acyl having 1-4 carbons, halogen, thiol, or thioketone;
X is COOH, tetrazole, $PO_3H$, $SO_3H$, CHO, $CH_2OH$, $CONH_2$, COSH, $COOR_9$, $COSR_9$, $CONHR_9$, or COOW where $R_9$ represents a lower alkyl having 1-4 carbons, phenyl, aromatic alkyl, or q-hydroxyphenyl, q-bromophenyl, q-chlorophenyl, q-fluorophenyl, or q-iodophenyl, where q=2-4, where W is a pharmaceutically acceptable salt;

One of Z, Z', Z'' and Z''' represents S, and the rest are C;
n=0-3; and
the dashed lines depict optional double bonds.

As used in this disclosure, pharmaceutically acceptable salts include but are not limited to: hydrochloric, hydrobromic, hydroiodic, hydrofluoric, sulfuric, citric, maleic, acetic, lactic, nicotinic, succinic, oxalic, phosphoric, malonic, salicyclic, phenylacetic, stearic, pyridine, ammonium, piperazine, diethylamine, nicotinamide, formic, urea, sodium, potassium, calcium, magnesium, zinc, lithium, cinnamic, methylamino, methanesulfonic, picric, tartaric, triethylamino, dimethylamino, and *tris* (hydroxymethyl) aminomethane. Additional pharmaceutically acceptable salts are known to those of skill in the art.

Typically said compound selectively activates Retinoid X Receptors in preference to Retinoid Acid Receptors.

Preferably said compound is of any of the following formulae:

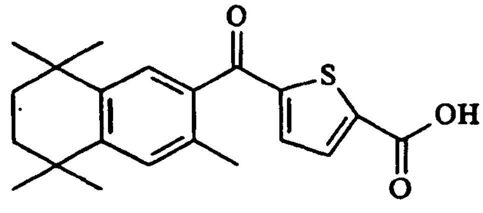

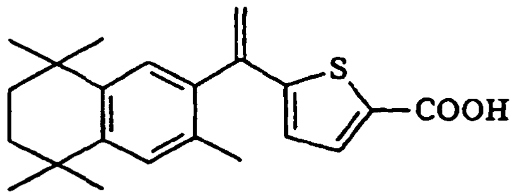

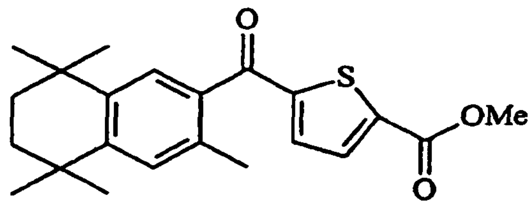

The present invention further provides a pharmaceutical composition comprising in a pharmaceutically acceptable vehicle suitable for enteral, parenteral, or topical administration, one or more compounds as described above.

The present invention further provides a method for modulating a process of *in vitro* cellular growth and differentiation mediated by one or more Retinoid X Receptors, said method comprising causing said process to be conducted in the presence of at least one compound described above.

Typically in said method the Retinoid X Receptor is Retinoid X Receptor-alpha, Retinoid X Receptor-beta, or Retinoid X Receptor-gamma.

The present invention further provides an *in vitro* method for modulating a process mediated by one or more Retinoid X Receptors, said method comprising causing said process to be conducted in the presence of at least one compound described above.

Additionally, the present invention provides a method for determining the presence of one or more Retinoid X Receptors comprising combining a compound as described above with a sample containing one or more unknown receptors and determining whether said ligand binds to any receptor in said sample.

Further provided by the present invention is a method of purifying Retinoid X Receptors, comprising combining a compound as described above, with a sample containing one or more said Retinoid X Receptors, allowing said compound to bind with Retinoid X Receptors, and separating out the bound combination of said compound and Retinoid X Receptor.

The present invention further provides a composition comprising a first ligand which selectively activates Retinoid X Receptors in preference to Retinoic Acid Receptors, in combination with a second ligand which selectively activates Retinoic Acid Receptors in preference to Retinoid X Receptors; the first ligand being a compound as described above.

Further provided by the present invention is a composition comprising a first ligand which selectively activates Retinoid X Receptors in preference to Retinoic Acid Receptors, in combination with a second ligand which activates one or more intracellular receptors other than Retinoid X Receptors; the first ligand being a compound as described above.

Typically, the physiological effect in mammals produced by said above-described composition, at a given concentration, is greater than the additive effect achieved utilizing each said ligand alone at said concentration.

Still further provided by the present invention is a pharmaceutical composition comprising in a pharmaceutically acceptable vehicle for enteral, parenteral, or topical administration a first ligand which selectively activates Retinoid X Receptors in preference to Retinoic Acid Receptors, in combination with a second ligand which selectively activates one or more intracellular receptors other than Retinoid X Receptors; the first ligand being a compound as described above.

Additionally provided is a pharmaceutical composition as described above wherein said second ligand selectively activates Retinoic Acid Receptors in preference to Retinoid X Receptors.

The present invention provides an *in vitro* method for modulating a process mediated by intracellular receptors, said method comprising causing said process to be conducted in the presence of a composition comprising a first ligand which selectively activates Retinoid X Receptors in preference to Retinoic Acid Receptors, in combination with a second ligand which activates one or more intracellular receptors other than Retinoid X Receptors, and wherein the physiological effect in mammals produced by said composition at a given concentration is greater than the additive effect achieved utilizing each said ligand alone at said concentration; the first ligand being a compound as described

above.

Preferably said composition is present at a concentration at which neither said first nor second ligand would alone produce a significant therapeutic response.

Typically, in the above-described method, said second ligand activates peroxisome proliferator activated receptors.

Typically, in the above-described method, said second ligand activates Vitamin D receptors.

Typically, in the above-described method, said second ligand activates thyroid hormone receptors, HNF4 receptors, or members of the COUP family of receptors.

The present invention also provides use of a compound as described above in the preparation of a medicament for the modulation of *in vivo* lipid metabolism, skin-related processes, programmed cell death, malignant cell development, premalignant lesions, autoimmune diseases or fatty acid metabolism.

The present invention further provides a compound as described above for administering to a mammalian subject to modulate a process mediated by one or more Retinoid X Receptors.

52 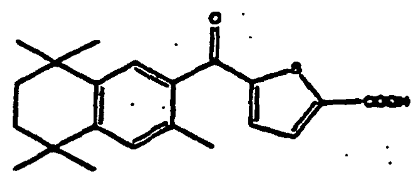

2-[(3,5,5,8,8-pentamethyl-5,6,7,8-tetrahydro-2-naphthyl)carbonyl] thiophene-5-carboxylic acid (TTNCTC)

53 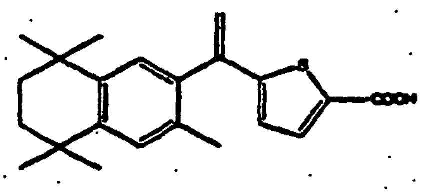

2-[1-(3,5,5,8,8-pentamethyl-5,6,7,8-tetrahydro-2-naphthyl)ethenyl] thiophene-5-carboxylic acid (TTNETC)

Representative derivatives of the present invention can be prepared according to the following illustrative synthetic schemes:

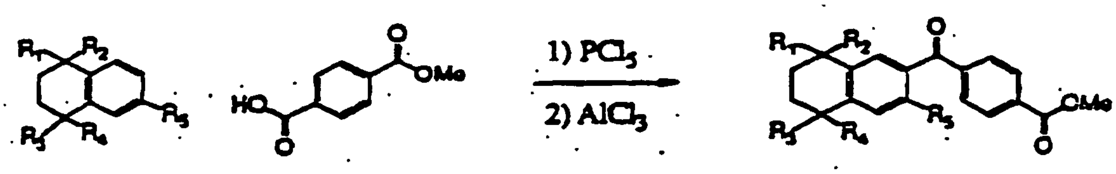


1 2 3

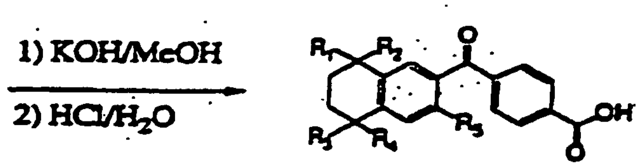


4

Compounds of structure 1 containing $R_5$ = lower alkyl are prepared in accordance with United States Patent No. 2,897,237. When $R_5$ = Halo, OH, amino, or thio, the products are prepared by standard Friedel-Crafts reaction

conditions combining the appropriate substituted benzene with 2,5-dichloro-2,5-dimethyl hexane in the presence of aluminum trichloride.

Condensation of 1 with mono-methyl terephthalate 2 was carried out by addition of $PCl_5$ to 1 and 2 in $CH_2Cl_2$ followed by addition of $AlCl_3$ at room temperature.

The resulting methyl esters 3 are hydrolyzed to the carboxylic acid 4 by refluxing in aqueous KOH-MeOH followed by acidification.

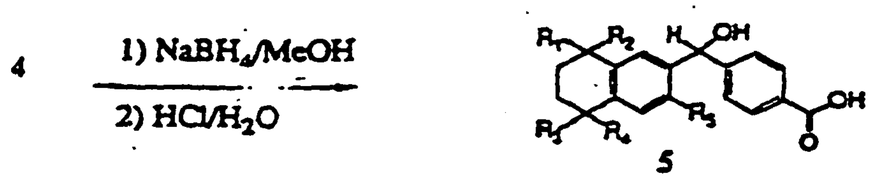


5

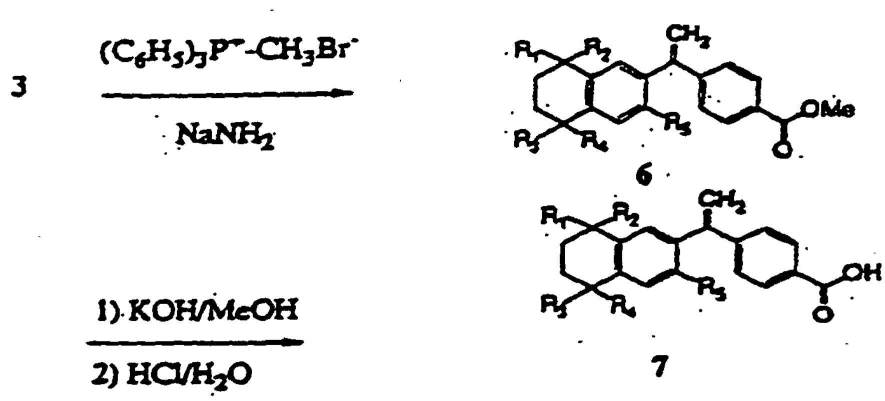


6

7

Treatment of ketone 4 with $NaBH_4$ afforded alcohol 5.

Treatment of the methyl ester 3 with methyltriphosphonium bromide-sodium amide in THF afforded the methano compound 6.

The carboxylic acid 7 was formed by adding KOH to methano compound 6 in MeOH, followed.by acidification.

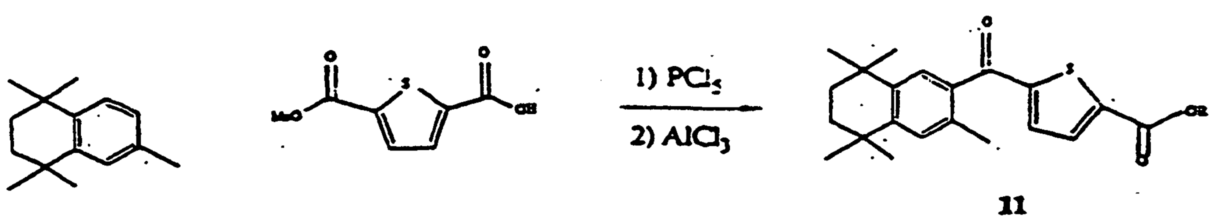


11

Condensation of 1 with thiophene 2,5-mono methyl dicarboxylic acid or furanyl 2,5-mono methyl dicarboxylic acid was carried out by addition of $PCl_5$ in $CH_2Cl_2$ followed by addition of $AlCl_3$ at room temperature to give esters 11 and 12, which were hydrolyzed with KOH followed by acidification to the corresponding acids.

Illustrative examples for the preparation of some of the compounds according to this invention are as follows:

Example 1

Preparation of compound 3 where $R_1$, $R_2$, $R_3$, $R_4$ and $R_5$ are methyl, R' and R" are oxo, and X=COOMe:

To 7 gm (34.7 mmol) of 1,1,4,4,6-pentamethyl-1,2,3,4-tetrahydronaphthalene and 6 gm (33.3 mmol) of mono-methyl teraphthalate in 200 mL of $CH_2Cl_2$ was added 8 g (38.8 mmol) of $PCl_5$. The reaction boiled vigorously and turned clear within 10 min. After stirring for an additional 1 h, 6 g (43.5 mmol) of $AlCl_3$ was added in 1 g portions over 15 min. and the reaction was allowed to stir overnight. The mixture was poured into 300 mL of 20% aqueous HCl and

extracted with 5% EtOAc-hexanes, dried (MgSO4), concentrated, and crystallized from MeOH to give ca. 6 gm (16.5 mmol) of methyl ester 3.
$^1$HNMR ($CD_3OCD_3$) δ 1.20 (s, 2($CH_3$)), 1.35 (s, 2($CH_3$)), 1.75 (s, 2($CH_2$)), 2.31 (s, $CH_3$), 3.93 (s, $COOCH_3$), 7.21 (s, Ar-CH), 7.23 (s, Ar-CH), 7.85 (d, J=8 Hz, Ar-2(CH)), 8.18 (d, J=8 Hz, Ar-2(CH)).

Example 2

Preparation of compound 4 where $R_1$, $R_2$, $R_3$, $R_4$ and $R_5$ are methyl, R' and R" are oxo, and X = COOH (3-methyl-TTNCB):

To 6 gm (16.5 mmol) of methyl ester 3 suspended in 100 mL of MeOH was added 50 mL of 5N aqueous KOH. The mixture was heated under reflux for 1 h., cooled, acidified (20% aqueous HCl) and the organics extracted with EtOAc. After drying (MgSO4), the product was concentrated and precipitated from 1:4 EtOAc-hexanes to give ca. 5 g (14.3 mmol) of acid 4.
$^1$HNMR ($CD_3OCD_3$) δ 1.20 (s, 2($CH_3$)), 1.35 (s, 2($CH_3$)), 1.75 (s, 2($CH_2$)), 2.31 (s, $CH_3$), 7.21 (s, Ar-CH), 7.23 (s, Ar-CH), 7.91 (d, J=8 Hz, Ar-2(CH)), 8.21 (d, J=8 Hz, Ar-2(CH)).

Example 3

Preparation of compound 5 where $R_1$, $R_2$, $R_3$, $R_4$ and $R_5$ are methyl, R' = H and R" = OH, and X = COOH (3-methyl-TTNHMB):

To a 1:1 THF-MeOH solution containing 1 g (2.86 mmol) of ketone 4 was added 100 mg of $NaBH_4$. The mixture was heated to 50°C for 10 min., cooled, acidified (20% aqueous HCl), and the organics extracted (EtOAc). After drying ($MgSO_4$), the product was concentrated and precipitated from 1:3 EtOAc-hexanes to give 550 mg (1.56 mmol) of the alcohol 5.
$^1$HNMR ($CD_3OCD_3$) δ 1.20 (s, $CH_3$)), 1.22 (s, ($CH_3$)), 1.22 (s, 2($CH_3$)), 1.65 (s, 2($CH_2$)), 2.21 (s, $CH_3$), 6.00 (s, -CHOH-), 7.09 (s, Ar-CH), 7.41 (s, Ar-CH), 7.53 (d, J=8 Hz, Ar-2(CH)), 8.01 (d, J=8 Hz, Ar-2(CH)).

Example 4

Preparation of compound 6 where $R_1$, $R_2$, $R_3$, $R_4$ and $R_5$ are methyl, R' and R" are methano, and X = COOMe:

To 1 gm of methyl ester 3 (2.7 mmol) in 25 mL of dry THF was added 1.2 g (3.08 mmol) of methyltriphosphonium bromide-sodium amide. The solution was stirred at RT for 3 h or until complete by TLC (20% EtOAc-hexanes). Water was added and the organics were extracted with EtOAc, dried ($MgSO_4$), concentrated and purified by $SiO_2$ chromatography (5% EtOAc-hexanes) followed by crystallization from MeOH to give 700 mg (1.93 mmol) of methano compound 6.
$^1$HNMR ($CD_3OCD_3$) δ 1.22 (s, 2($CH_3$)), 1.30 (s, 2($CH_3$)), 1.72 (s, 2($CH_2$)), 1.95 (s, $CH_3$), 3.85 (s, $COOCH_3$), 5.29 (s, =CH), 5.92 (s, =CH), 7.19 (s, Ar-CH), 7.20 (s, Ar-CH), 7.39 (d, J=8 Hz, Ar-2(CH)), 7.96 (d, J=8 Hz, Ar-2 (CH)).

Example 5

Preparation of compound 7 where $R_1$, $R_2$, $R_3$, $R_4$ and $R_5$ are methyl, R' and R" are methano, and X = COOH (3-methyl-TTNEB):

To 500 mg of methano compound 6 (1.38 mmol) in 20 mL of MeOH was added 5 mL of 5 N aqueous KOH and the suspension was refluxed for 1 h. After acidification (20% aqueous HCl) the organics were extracted (EtOAc), dried ($MgSO_4$), concentrated, and the solids recrystallized from EtOAc-hexanes 1:5 to give 350 mg (1.0 mmol) of the carboxylic acid 7.
$^1$HNMR ($CD_3OCD_3$), δ 1.22 (s, 2($CH_3$)), 1.30 (s, 2($CH_3$)), 1.72 (s, 2($CH_2$)), 1.95 (s, $CH_3$), 5.22 (s,=CH), 5.89 (s,=CH), 7.19 (s, Ar-CH), 7.20 (s, Ar-CH), 7.39 (d, J=8 Hz, Ar-2(CH)), 7.96 (d, J = 8 Hz, Ar-2(CH)).

Example 6

Preparation of compound 52 where $R_1$, $R_2$, $R_3$, $R_4$, $R_5$ are methyl, R' and R" = oxo, Z = S, and X = COOH (TTNCTC):

To 1 g (4.9 mmol) of 1,1,4,4,6-pentamethyl-1,2,3,4-tetrahydro-naphthalene and 1 g (4.9 mmol) of mono methyl thiophene carboxylic acid chloride in 25 mL of $CH_2Cl_2$ was added 1 g (7.5 mmol) of $AlCl_3$. The reaction was heated to reflux for 15 m followed by cooling and addition of 20% aqueous HCl. The product was extracted with EtOAc, washed ($H_2O$, brine), dried ($MgSO_4$), filtered, concentrated, and purified by crystallization from MeOH to give 450 mg (1.21 mmol) of the methyl ester of 52 (25% yield). The methyl ester was hydrolized in methanolic KOH followed by acidification

(20% HCl) extraction with EtOAc, washed ($H_2O$, brine), dried ($MgSO_4$), filtered, concentrated, and purified by crystallization from EtOAc-hexane to give 375 mg (1.05 mmol) of 52 (87% yield). MP: 206 °C; $^1$H-NMR ($CDCl_3$) δ1.26 (s,2 ($CH_3$)), 1.31 (s,2($CH_3$)), 1.71 (s,2($CH_2$)), 2.38 (s,$CH_3$), 7.21 (s,Ar-CH), 7.44 (s,Ar-CH), 7.48 (d,J=4 Hz, Thio Ar-CH), 7.85 (d,J=4 Hz,ThioAr-CH).

Example 7

Preparation of compound 53 where $R_1$,$R_2$,$R_3$,$R_4$,$R_5$ are methyl, R' and R" = methano, Z = S, and X = COOH (TTNETC):

Compound 53 was prepared from the methyl ester of 52 in a manner similar to examples 4 and 5. MP: 200 °C; $^1$H-NMR ($CDCl_3$) δ1.26 (s,2($CH_3$)), 1.30 (s,2($CH_3$)), 1.69 (s,2($CH_2$)), 2.10. (s,$CH_3$), 5.21 (s,=CH), 5.88 (s,=CH), 6.76 (d,J=4 Hz,Thio Ar-CH), 7.11 (s,Ar-CH), 7.23 (s,Ar-CH), 7.68 (d,J=4 Hz,Thio Ar-CH).

Representative synthetic retinoid compounds of the current invention were analyzed and found to exhibit subtype selectivity for retinoid receptors, and to be capable of modulating processes selectively mediated by retinoid X receptors, as discussed more fully below.

As employed herein, the phrase "processes selectively mediated by retinoid X receptors" refers to biological, physiological, endocrinological, and other bodily processes which are mediated by receptors or receptor combinations which are responsive to retinoid X receptor selective processes, *e.g.*, compounds which selectively activate one and/or multiple members of the RXR subfamily. Modulation of such processes can be accomplished *in vitro* or *in vivo*. *In vivo* modulation can be carried out in a wide range of subjects, such as, for example, humans, rodents, sheep, pigs, cows, and the like.

The receptors which are responsive to retinoid X receptor selective ligands include: retinoid X receptor-alpha, retinoid X receptor-beta, retinoid X receptor-gamma, and splicing variants encoded by the genes for such receptors, as well as various combinations thereof (*i.e.*, homodimers, homotrimers, heterodimers, heterotrimers, and the like). Also included are combinations of retinoid X receptors with other members of the steroid/thyroid superfamily of receptors with which the retinoid X receptors may interact by forming heterodimers, heterotrimers, and the higher heteromultimers. For example, the retinoic acid receptor-alpha, -beta, or -gamma isoforms form a heterodimer with any of the retinoid X receptor isoforms, (*i.e.*, alpha, beta, or gamma, including any combination of the different receptor isoforms), and the various retinoid X receptors form a heterodimer with thyroid receptor and form a heterodimer with vitamin D receptor. Members of the retinoid X receptor subfamily form a heterodimer with certain "orphan receptors" including PPAR (Issemann and Green, *Nature,* 347:645-49 (1990)); HNF4 (Sladek *et al., Genes & Development* 4:2353-65 (1990)); the COUP family of receptors (*e.g.,* Miyajima *et al.*, *Nucleic Acids Research* 16:11057-74 (1988), and Wang *et al., Nature*, 340:163-66 (1989)) ; COUP-like receptors and COUP homologs, such as those described by Mlodzik *et al.* (*Cell*, 60:211-24 (1990)) and Ladias *et al. (Science*, 251:5561-65 (1991)); the ultraspiracle receptor (*e.g.*, Oro *et al., Nature,* 347:298-301 (1990)); and the like.

As employed herein, the phrase "members of the steroid/thyroid superfamily of receptors" (also known as "nuclear receptors" or "intracellular receptors") refers to hormone binding proteins that operate as ligand-dependent transcription factors. Furthermore, this classification includes identified members of the steroid/thyroid superfamily of receptors for which specific ligands have not yet been identified (referred to hereinafter as "orphan receptors'). All members of the intracellular receptor superfamily have the intrinsic ability to bind to specific DNA sequences. Following binding, the transcriptional activity of a target gene (*i.e.*, a gene associated with the specific DNA sequence) is modulated as a function of the ligand bound to the receptor. Also, see Heyman *et al., Cell*, 68:397-406 (1992), and copending U.S. Serial No. 809,980, filed December 18, 1991.

The modulation of gene expression by the ligand retinoic acid and its receptors can be examined in a reconstituted system in cell culture. Such a system was used to evaluate the synthetic retinoid compounds of this invention for their interaction with the retinoid receptor subtypes RARα, RARβ, RARγ, RXRα, RXRβ, and RXRγ.

The system for reconstituting ligand-dependent transcriptional control, which was developed by Evans *et al.*, *Science*, 240:889-95 (1988), has been termed a "co-transfection" or "*cis-trans*" assay. This assay is described in further detail in U.S. Patent Nos. 4,981,784 and 5,071,773. Also see Heyman *et al.*, *Cell*, 68:397-406 (1992). The co-transfection assay provides a mechanism to evaluate the ability of a compound to modulate the transcription response initiated by an intracellular receptor. The co-transfection assay is a functional, rapid assay that monitors hormone or ligand activity and is a good predictor of an *in vivo* system.

Briefly, the co-transfection assay involves the introduction of two plasmids by transient transfection into a retinoid receptor-negative mammalian cell background. The first plasmid contains a retinoid receptor cDNA and directs constitutive expression of the encoded receptor. The second plasmid contains a cDNA that encodes for a readily quantifiable protein, *e.g.*, firefly luciferase or chloramphenicol acetyl transferase (CAT), under control of a promoter containing a retinoid acid response element, which confers retinoid dependence on the transcription of the reporter. In this co-transfection assay, all retinoid receptors respond to all-*trans*-retinoic acid in a similar fashion. This assay can

be used to accurately measure efficacy and potency of retinoic acid and synthetic retinoids as ligands that interact with the individual retinoid receptor subtypes.

Accordingly, synthetic retinoid compounds of the current invention were evaluated for their interaction with retinoid receptor subtypes using the co-transfection assay in which CV-1 cells were co-transfected with one of the retinoid receptor subtypes, a reporter construct, and an internal control to allow normalization of the response for transfection efficiency.

In addition to compounds disclosed above, the compounds below are referred to in the following illustrative examples.

3-methyl-TTNCB

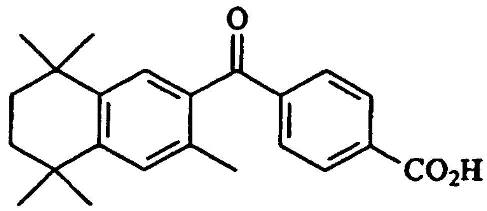

3-methyl-TTNEB

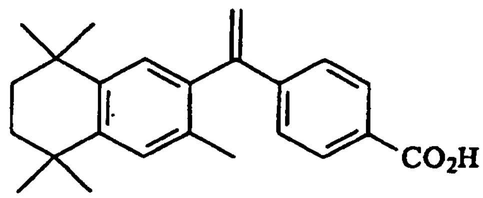

3-bromo-TTNEB

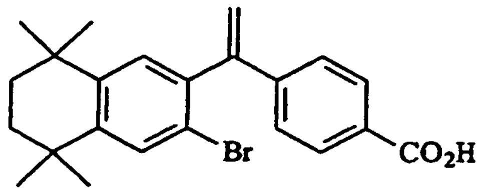

3-methyl-TTNCHBP

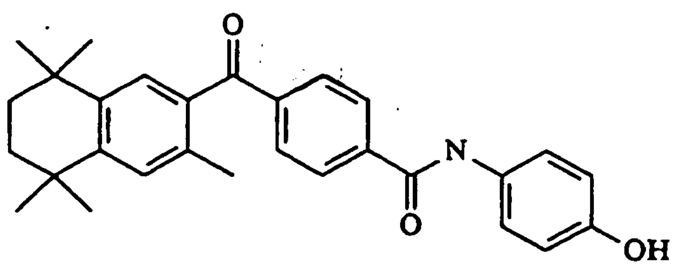

3-methyl-TTNEHBP

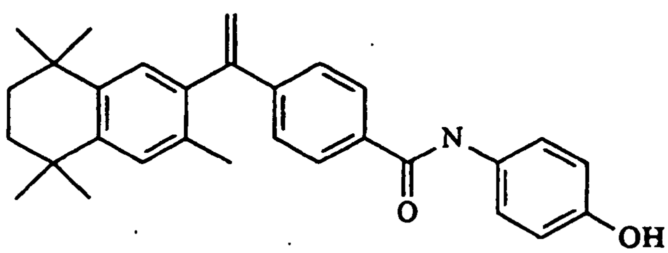

TPNCP

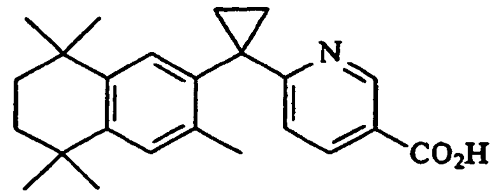

TPNEP

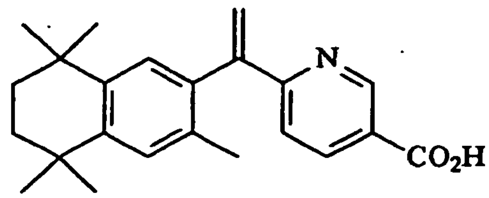

The following example is illustrative.

Illustrative Example 1

Retinoids: All-*trans*-retinoic acid (RA) and 13-*cis*-retinoic acid (13-*cis*-RA) were obtained from Sigma. 9-*cis*-retinoic acid (9-*cis*-RA) was synthesized as described in Heyman *et al*., *Cell*, 68:397-406 (1992). Retinoid purity was established as greater than 99% by reverse phase high-performance liquid chromatography. Retinoids were dissolved in dimethylsulfoxide for use in the transcriptional activation assays.

Plasmids: The receptor expression vectors used in the co-transfection assay have been described previously (pRShRAR-α: Giguere *et al*. (1987); pRShRAR-β and pRShRAR-γ: Ishikawa *et al*. (1990); pRShRXR-α: Mangelsdorf *et al*., (1990); pRSmRXR-β and pRSmRXR-γ: Mangelsdorf *et al*., *Genes & Devel*., 6:329-44 (1992)). A basal reporter plasmid Δ-MTV-LUC (Hollenberg and Evans, *Cell*, 55:899-906 (1988)) containing two copies of the TRE-palindromic response element 5'-TCAGGTCATGACCTGA-3' (Umesono *et al*., *Nature*, 336:262-65 (1988)) was used in transfections for the RARs, and CRBPIIFKLUC, which contains an RXRE (retinoid X receptor response element (Mangelsdorf *et al*., *Cell*, 66:555-61 (1991)), was used in transfections for the RXRs.

Co-transfection Assay In CV-1 Cells: A monkey kidney cell line, CV-1, was used in the *cis-trans* assay. Cells were transfected with two plasmids. The trans-vector allowed efficient production of the retinoid receptor in these cells, which do not normally express this receptor protein. The *cis*-vector contains an easily assayable gene product, in this case the firefly luciferase, coupled to a retinoid-responsive promoter, *i.e*., an RARE or RXRE. Addition of retinoic acid or an appropriate synthetic retinoid results in the formation of a retinoid-RAR or - RXR complex that activates the expression of luciferase gene, causing light to be emitted from cell extracts. The level of luciferase activity is directly proportional to the effectiveness of the retinoid-receptor complex in activating gene expression. This sensitive and reproducible co-transfection approach permits the identification of retinoids that interact with the different receptor isoforms.

Cells were cultured in DMEM supplemented with 10% charcoal resin-stripped fetal bovine serum, and experiments were conducted in 96-well plates. The plasmids were transiently transfected by the calcium phosphate method (Umesono and Evans, *Cell*, 57:1139-46 (1989) and Berger *et al*., *J. Steroid Biochem. Molec. Biol*., 41:733-38 (1992)) by using 10 ng of a pRS (Rous sarcoma virus promoter) receptor-expression plasmid vector, 50 ng of the reporter luciferase (LUC) plasmid, 50 ng of pRSβ-GAL(β-galactosidase) as an internal control, and 90 ng of carrier plasmid, pGEM. Cells were transfected for 6 h and then washed to remove the precipitate. The cells were then incubated for 36 h with or without retinoid. After the transfection, all subsequent steps were performed on a Beckman Biomek Automated Workstation. Cell extracts were prepared, then assayed for luciferase and β-galactosidase activities, as described by Berger *et al*. (1992). All determinations were performed in triplicate in two independent experiments and were normalized for transfection efficiency by using β-galactosidase as the internal control. Retinoid activity was normalized relative to that of all-*trans*-retinoic acid and is expressed as potency (EC50), which is the concentration of retinoid required to produce 50% of the maximal observed response, and efficacy (%), which is the maximal response observed relative to that of all-*trans*-retinoic acid at $10^{-5}$M. The data obtained is the average of at least four independent experiments. Efficacy values less than 5% are not statistically different than the 0% background. Compounds with an efficacy of less than 20% at concentrations of $10^{-5}$ M are considered to be inactive. At higher concentrations of compound, such as $10^{-4}$ M, these compounds are generally toxic to cells and thus the maximal efficacy at $10^{-5}$ M is reported

in the tables and figures contained herein.

The synthetic retinoid compound 3-methyl-TTNCB, as described above, was evaluated for its ability to regulate gene expression mediated by retinoid receptors. As shown in Figure 1, this compound is capable of activating members of the RXR subfamily, *i.e.*, RXRα, RXRβ, and RXRγ, but clearly has no significant activity for members of the RAR subfamily, *i.e.*, RARα, RARβ, and RARγ. Assays using all-*trans*-retinoic acid (Figure 2) and 9-*cis*-retinoic acid (Figure 3) were run for reference, and demonstrate that these retinoic acid isomers activate members of both the RAR and RXR subfamilies.

Potency and efficacy were calculated for the 3-methyl-TTNCB compound, as summarized in the following table. For reference, the data for 9-*cis*-retinoic acid are also included.

TABLE 1

| | Potency (nM) | Efficacy |
|---|---|---|
| 3-Methyl-TTNCB | | |
| RXRα | 330 | 130% |
| RXRβ | 200 | 52% |
| RXRγ | 260 | 82% |
| RARα | >10,000 | <2% |
| RARβ | >10,000 | <4% |
| RARγ | >10,000 | <4% |
| 9-cis-retinoic acid | | |
| RXRα | 150 | 140% |
| RXRβ | 100 | 140% |
| RXRγ | 110 | 140% |
| RARα | 160 | 100% |
| RARβ | 5 | 82% |
| RARγ | 47 | 120% |

As shown by the data in Table 1, 3-methyl-TTNCB readily and at low concentrations activates RXRs. Further, 3-methyl-TTNCB is more potent an activator of RXRs than RARs, and preferentially activates RXRs in comparison to RARs, in that much higher concentrations of the compound are required to activate the RARs. In contrast, 9-*cis*-retinoic acid does not preferentially activate the RXRs, as also shown in Table 1. Rather, 9-*cis*-retinoic acid activates the RARβ and RARγ isoforms at lower concentrations and more readily than the RXRβ and RXRγ isoforms, and has substantially the same, within the accuracy of the measurement, activity for the RARα isoform in comparison to the RXRα isoform.

An extract reported to contain 9-*cis*-retinoic acid has previously been reported as at least 10-fold more potent in inducing RXRα than RARα (Heyman *et al.*, *Cell*, 68:397,399 (January 24, 1992)). Presently available data indicate that 9-*cis*-retinoic acid does not preferentially activate RXRs in comparison to RARs, as shown and discussed above. The compounds of this invention preferentially activate RXRs in comparison to RARs, and are preferably at least three times more potent as activators of RXRs than RARs, and more preferably at least five times more potent as activators of RXRs than RARs.

Potency and efficacy have also been calculated for the 3-methyl-TTNEB, 3-bromo-TTNEB, 3-methyl-TTNCHBP, 3-methyl-TTNEHBP, TPNEP, and TPNCP compounds, as summarized below in Table 2.

TABLE 2

| | Potency (nM) | Efficacy |
|---|---|---|
| 3-Methyl-TTNEB | | |
| RXRα | 40 | 83% |
| RXRβ | 21 | 102% |
| RXRγ | 34 | 80% |
| RARα | >10,000 | 6% |

TABLE 2 (continued)

| | Potency (nM) | Efficacy |
|---|---|---|
| 3-Methyl-TTNEB | | |
| RARβ | >10,000 | 17% |
| RARγ | >10,000 | 19% |
| 3-Bromo-TTNEB | | |
| RXRα | 64 | 88% |
| RXRβ | 54 | 49% |
| RXRγ | S2 | 71% |
| RARα | >10,000 | 3% |
| RARβ | >10,000 | 18% |
| RARγ | >10,000 | 15% |
| 3-Methyl-TTNCHBP | | |
| RXRα | 1100 | 113% |
| RXRβ | 1100 | 155% |
| RXRγ | 300 | 128% |
| RARα | >10,000 | <2% |
| RARβ | >10,000 | 7% |
| RARγ | >10,000 | 17% |
| 3-Methyl-TTNEHBP | | |
| RXRα | 140 | 125% |
| RXRβ | 71 | 121% |
| RXRγ | 48 | 163% |
| RARα | >10,000 | <2% |
| RARβ | 1,900 | 25% |
| RARγ | >10,000 | 10% |
| TPNEP | | |
| RXRα | 5 | 75% |
| RXRβ | 5 | 138% |
| RXRγ | 6 | 100% |
| RARα | >10,000 | <2% |
| RARβ | >10,000 | <2% |
| RARγ | 1,500 | 24% |
| TPNCP | | |
| RXRα | 4 | 63% |
| RXRβ | 4 | 93% |
| RXRγ | 3 | 49% |
| RARα | >10,000 | <2% |
| RARβ | >10,000 | <2% |
| RARγ | >10,000 | <2% |

As shown by the data in Table 2, 3-methyl-TTNEB, 3-bromo-TTNEB, 3-methyl-TTNCHBP, 3-methyl-TTNEHBP, TPNEP, and TPNCP each readily and preferentially activate the RXRs, and are more potent as activators of RXRs than of RARs. The diminished activity of these compounds for the RARs in comparison to the RXRs is also shown for some of these compounds in Figures 4-7.

It can be expected that synthetic retinoid ligands, such as those exemplified in Tables 1 and 2 which preferentially affect some but not all of the retinoic acid receptor isoforms, can, in pharmacological preparations, provide pharmaceuticals with higher therapeutic indices and a better side effect profile than currently used retinoids. For example, the compounds of the present invention have been observed to be less irritating to the skin than standard retinoids.

The retinoid compounds of this invention are useful for the treatment of certain dermatological conditions such as keratinization disorders, *i.e.*, differentiation/proliferation. A standard assay to determine the activity of these compounds is the measurement of the enzymatic activity for transglutaminase; this is a measure of the antiproliferative action, of retinoids. Retinoids have been shown to inhibit the pathway of differentiation, which is indicated by a decrease in several biochemical markers that are associated with the expression of squamous cell phenotype, such as transglutaminase. (Yuspa *et al., Cancer Research*, 43:5707-12 (1983)). As can be seen from Figure 8, the 3-methyl-TTNCB compound is capable of inhibiting transglutaminase activity and inhibits 50% of the enzyme activity at $1 \times 10^{-7}$ M.

The compounds of this invention also exhibit good comedolytic activity in the test on Rhino mice described by Kligman *et al. (J. of Inves. Derm.*, 73:354-58 (1979)) and Mezick *et al. (J. of Inves. Derm.*, 83:110-13 (1984)). The test on Rhino mice has been a model for screening comedolytic agents. The activity of the 3-methyl-TTNCB retinoid compound, as well as 9-*cis* and all-*trans* retinoic acid is shown in Figure 9. A 0.1% solution of 3-methyl-TTNCB is capable of inhibiting the utriculi diameter by approximately 50%. It has also been observed that 3-methyl-TTNCB is less irritating to the skin of Rhino mice than 9-*cis*- or all-*trans*-retinoic acid.

The synthetic retinoids of the current invention have also been tested using radioligand displacement assays. RAR and RXR isoforms overexpressed in *E. coli* or baculovirus are capable of binding radiolabeled 9-*cis*-retinoic acid with binding parameters which are essentially similar to those receptors overexpressed in mammalian cells. By testing the abilities of various synthetic retinoids to compete with the radiolabeled retinoic acid for binding to various receptor isoforms, the relative dissociation constant for the receptor itself can be determined. This is an important supplementary analysis to the co-transfection assay since it can detect important discrepancies that may arise due to the various determinants of retinoid activity in the co-transfection assay. These determinants may include (1) activating or inactivating metabolic alterations in the test compounds, (2) binding to serum proteins which alter the free concentration of the test compound, (3) differences in cell permeation among test compounds, (4) intrinsic differences in the affinity of the test compounds for the receptor proteins, *i.e.*, in $K_d$, and (5) conformational changes produced in the receptor after binding of the test compound, reflected in the effects on reporter gene expression.

The 3-methyl-TTNCB compound is capable of displacing $^3$H-9-*cis*-retinoic acid bound to the RXRs, but is not capable of displacing radiolabeled ligand that is bound to the RARs. This indicates that the 3-methyl-TTNCB compound preferentially binds RXRs in comparison to RARs, a property which would be expected of a ligand selective for the RXRs.

It has been recognized that the co-transfection assay provides a functional assessment of the ligand being tested as either an agonist or antagonist of the specific genetic process sought to be affected. Ligands which do not significantly react with other intracellular receptors, as determined by the co-transfection assay, can be expected to result in fewer pharmacological side effects. Because the co-transfection assay is run in living cells, the evaluation of a ligand provides an early indicator of the potential toxicity of the candidate at concentrations where a therapeutic benefit would be expected.

Processes capable of being modulated by retinoid receptors, in accordance with the present invention, include *in vitro* cellular differentiation, the regulation of morphogenetic processes including limb morphogenesis, regulation of cellular retinol binding protein (CRBP), and the like. As readily recognized by those of skill in the art, the availability of ligands for the retinoid X receptor makes it possible, for the first time, to elucidate the processes controlled by members of the retinoid X receptor subfamily. In addition, it allows development of assays for the identification of antagonists for these receptors.

The processes capable of being modulated by retinoid receptors, in accordance with the present invention, further include the *in vivo* modulation of lipid metabolism; *in vivo* modulation of skin related processes (*e.g.*, acne, psoriasis, aging and wrinkling; *in vivo* modulation of programmed cell death (a poptosis); *in vivo* modulation of malignant cell development, such as occurs, for example, in acute promyelocytic leukemia, mammary cancer, prostate cancer, lung cancer, cancers of the aerodigestive pathway, skin cancer, bladder cancer, and sarcomas; *in vivo* modulation of premalignant lesions, such as occurs with oral leukoplakia ; *in vivo* modulation of auto-immune diseases such as rheumatoic arthritis; and *in vivo* modulation of fatty acid metabolism. Such applications can be expected to allow the modulation of various biological processes with reduced occurrence of undesirable side effects such as teratogenic effects, skin irritation, mucosal dryness and lipid disturbances. *In vivo* applications can be employed with a wide range of

subjects, such as, for example, humans, rodents, sheep, pigs, and cows.

For example, regarding the *in vivo* modulation of lipid metabolism referred to above, apolipoprotein AI is a major protein component of plasma high density lipoprotein (HDL) cholesterol. Since the circulating level of HDL in humans has been shown to be inversely correlated to the risk of coronazy vascular diseases, it can be expected that regulating synthesis of apolipoprotein AI can be utilized in the treatment of coronary vascular disease. It has been established that regulation of transcription of apolipoprotein AI is controlled by members of the intra-cellular receptor superfamily, and further that the apolipoprotein AI gene transcription start site A is a highly selective retinoid responsive element (RXRE) that responds preferentially to RXRα. Rottman *et al., Mol. Cell. Biol.*, 11:3814-20 (1991). Therefore, ligands which selectively activate members of the RXR family of retinoic acid receptors may regulate apolipoprotein AI transcription. We have demonstrated in *in vivo* studies that ligands having selective activity for RXRs can be used to significantly raise plasma HDL levels, as demonstrated in the following example.

Illustrative Example 2

Male Sprague-Dawley rats (160-200 gram) were obtained from Harland. Animals were fed standard laboratory diets (Harlan/Teklad) and kept in an environmentally controlled animal house with a light period lasting from 6 a.m. to 6 p.m. Animals were treated with drugs prepared as suspensions in olive oil.

To verify that RXR activation can modulate HDL cholesterol, an initial study was carried out that included dosing rats for 4 days with an RAR-selective compound, all-*trans* retinoic acid, the non-selective RAR/RXR agonist, 9-*cis*-retinoic acid, and either of two RXR-selective agents, 3-methyl-TTNCB or 3-methyl-TTNEB. Each drug was administered at a dose of 100 mg/kg, i.p. Positive control groups received olive oil as a vehicle. Twenty-four hours after the last treatment, rats were sacrificed by $CO_2$ inhalation, blood was collected from the inferior vena cava into a tube containing 0.1 ml of 0.15% EDTA and centrifuged at 1500 x g for 20 min. at 4°C. Plasma was separated and stored at 4°C for evaluation of plasma total cholesterol and high density lipoprotein cholesterol (HDL-cholesterol).

Plasma total cholesterol was measured enzymatically utilizing Boeringer Mannheim Diagnostics High Performance Cholesterol Methods with an ABBOTT VP Bichromatic Analyzer. HDL was measured after preparation of the HDL-containing fraction by heparin-manganese precipitation of plasma. HDL-cholesterol in this fraction was estimated as mentioned earlier. All HDL separations were checked for contamination by lipoproteins with agarose gel electrophoresis.

The results of this study are shown in Figure 10. As shown, rats receiving the RXR-selective compounds exhibited substantial and statistically significant increases in HDL levels, particularly when receiving 3-methyl-TTNEB. Because the RXR-selective ligand 3-methyl-TTNEB was the most efficacious, additional 4 day experiments were conducted with this agent at doses of 0.3, 1, 3, 6, 10, 30, 100, or 300 mg/kg i.p. in 0.5 ml olive oil or 1, 3, 10, 30, 100, 300 mg/kg p.o. in 0.5 ml olive oil for 4 days. An additional 30 day p.o. study was conducted with 10, 30, or 100 mg/kg 3-methyl-TTNEB to determine whether tolerance would develop to its pharmacological actions. For the rats receiving 3-methyl-TTNEB in various doses for four days, it was also observed that most of the HDL elevation was obtainable with relatively low doses (less than 5 mg/kg) of 3-methyl-TTNEB. The 30-day study with 3-methyl-TTNEB did not indicate development of tolerance to its pharmacological action.

Additional *in vitro* studies were also performed utilizing the co-transfection assay previously described within this application to demonstrate the effect of RXR-selective ligands on regulation of transcription of apolipoprotein-AI, as described in the following example.

Illustrative Example 3:

This work focused on studying the transcriptional properties of the retinoid receptors RAR. and RXR on a reporter molecule (*e.g.*, luciferase) under control of a basal promoter containing the RXR response element from the apolipoprotein AI gene ("A" site). Plasmid constructs coding for the various receptors were transfected into a human hepatocyte cell line (HepG-2) along with the reporter plasmid. Reporter plasmids-contained multimets of the apolipoprotein-AI "A" site (-214 to -192 relative to transcription start site) shown to bind RXR. Widom *et al.*, *Mol. Cell. Biol.* 12: 3380-89 (1992); Ladias & Karathanasis, *Science* 251:561-65 (1991). After transfection, treatment, harvest, and assay, the data obtained was normalized to transfected beta-galactosidase activity so as to control for transfection efficiency. The results demonstrated activation in the system with the RXR-specific ligands 3-methyl-TTNCB and 3-methyl-TTNEB, demonstrating that the RXR specific ligands could regulate the transcriptional properties via the "A" site from the apolipoprotein AI gene. These compounds had no effect when RAR was used in the transfection, demonstrating receptor specificity. The transcriptional regulation by RXR was dependent on the presence of the hormone response element.

It has been surprisingly found that the administration of a compound containing a ligand which has specific activity for RXRs but essentially no activity for RARs, in combination with a ligand that has specific activity for RARS

but not RXRs, provides a cellular response at extremely low dosages, dosages at which the ligands individually provide no significant response. Specifically, the concentration-related effect of an RXR-specific ligand and a RAR-specific ligand on proliferation of a myeloma cell line (RPMI 8226) was studied in *in vitro* studies using a thymidine incorporation assay. (L.M. Bradley, Selected Methods in Cellular Immunology, Ch. 10.1, pp. 235-38, Mishell & Shiigi (eds.), Freeman & Co., New York, 1980). This assay examines the incorporation of radiolabeled thymidine into DNA, and by determining the ability of a compound to inhibit thymidine incorporation into DNA, provides a measure of cell proliferation. Compounds which inhibit cell proliferation have well-known utility in the treatment of certain cancers.

As shown previously (Table 2), 3-methyl-TTNEB activates members of the RXR subfamily and has no significant activity for members of the RAR subfamily. Examination of the effects of 3-methyl-TTNEB on the proliferation of myeloma cells show a concentration dependent inhibition of thymidine incorporation. The $IC_{50}$ (the concentration of 3-methyl-TTNEB required to produce 50% inhibition of the maximal response) is $10^{-7}$ M, as shown in Figure 11. Concentrations less than $10^{-8}$ M provide essentially no effect on cell proliferation, as also shown in Figure 11.

It is well known that the compound TTNPB activates members of the RAR subfamily and has no significant activity for members of the RXR subfamily. The compound TTNPB is shown below, and its activity is shown in Table 3.

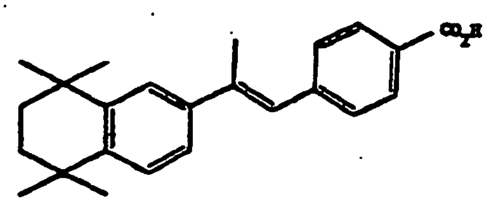

TTNPB

TABLE 3

| | Potency (nM) | Efficacy |
|---|---|---|
| TTNPB | | |
| RXRα | >10,000 | <5% |
| RXRβ | >10,000 | <5% |
| RXRγ | >10,000 | <5% |
| RARα | 52 | 30 |
| RARβ | 4 | 40 |
| RARγ | 0.4 | 50 |

The effect of TTNPB on cell proliferation is shown in Figure 11. The $IC_{50}$ value of TTNPB is about 5 x $10^{-11}$ M, and a concentration of less than $10^{-11}$ M produces essentially no effect on cell proliferation.

However, it has been found that when these two compounds (3-methyl-TTNEB and TTNPB) are present together, each at a concentration where the compound alone produces substantially no anti-proliferative effect, the combination of the two compounds effectively blocks cell proliferation. The combination of the two compounds appears to produce a greater than additive, or synergistic, effect.

For example, as shown in Figure 12, the presence of TTNPB at a concentration of $10^{-11}$ M produces a 9% inhibition on thymidine incorporation. However, combining it with 3-methyl-TTNEB at a concentration of $10^{-8}$ M (which results in no effect on cell proliferation) produces a greatly enhanced inhibitory effect of 49%. Likewise, it has also been found that the inhibitory effect of 3-methyl-TTNEB is greatly increased by the presence of TTNPB at a concentration which alone produces no effect.

Since it is well-known that toxic side effects of compounds such as TTNPB are concentration-dependent, the synergistic effect resulting from combining such RAR-specific compounds with RXR-specific compounds can be expected to permit lower dosages that are efficacious and to therefore reduce toxic side effects. For example, in cancer chemotherapy, use of two such compounds, in combination, at relatively low doses can be expected to produce the desired beneficial effect, while minimizing undesired side effects which result at higher doses of the compounds.

*In vitro* studies utilizing the co-transfection assay have also shown this same synergistic effect. For example, utilizing the co-transfection assay described previously and employing RAR-α and RXR-α and a reporter consisting of the ApoAI response element "A" site in the context of TKLUC (Ladias & Karathanasis, *Science* 251:561-65 (1991),

transfections were performed in HEPG2 cells. In this study, 100ng of the designated receptor were used and RSVCAT was used as a carrier to keep the amount of RSV promoter constant. All compounds were added at a final concentration of $10^{-7}$ M. The RXR specific compound, 3-methyl-TTNEB (Table 2, above), and the RAR specific compound, TTNPB (Table 3, above) were utilized. As shown below in Table 4, the relative normalized response observed utilizing the co-transfection assay also demonstrated a synergistic effect when a combination of the two compounds was utilized, compared to the response achieved utilizing the compounds individually.

TABLE 4

| Compound | Reporter Activity (Fold Induction) |
|---|---|
| 3-methyl-TTNEB | |
| TTNPB | 3 |
| 3-methyl-TTNEB + TTNPB | 71 |

As will be discernable to those skilled in the art from the foregoing discussions, the biological response of an RAR selective compound at a given concentration can be synergistically enhanced by combining the compound with an RXR selective compound. Similarly, the biological response of an RXR selective compound can be enhanced by combining the compound with an RAR selective compound. Thus, it becomes possible to achieve a desirable biological response, using a combination of PAR and RXR selective compounds, at lower concentrations than would be the case using the compounds alone. Among the advantages provided by such combinations of RAR and RXR selective compounds are desirable therapeutic effects with fewer side effects. In addition, novel effects that are not obtainable with either agent alone may be achieved by combinations of RAR and RXR selective compounds.

It has been further demonstrated that RXR-specific compounds also synergistically enhance the response of other hormonal systems. Specifically, peroxisome proliferator-activated receptor (PPAR) is a member of the intracellular receptor super family that plays a role in the modulation of lipid homeostasis. PPAR has been shown to be activated by amphipathic carboxylates, such as clofibric acid, and these agents, called peroxisome proliferators, have been used in man as hypolipidemic agents. The addition of 9-cis-retinoic acid (a retinoid ligand which activates both RAR and RXR receptors) and clofibric acid to HepG2 cells transfected with RXR$\alpha$ and PPAR expression plasmids, results in the activation of receptor gene which was greater than the sum of the activation with each ligand separately. (Kliewer *et al., Nature* 358:771 (1992)). Similarly, when the above two receptors were co-transfected into HepG2 cells, the addition of both an RXR-specific ligand (3-methyl-TTNEB) and clofibric acid was found to produce a greater than additive response as determined by activation of a target reporter gene, as shown below in Table 5.

TABLE 5

| Compound | Normalized Response (%) |
|---|---|
| clofibric Acid | 100 |
| 3-methyl-TTNEB | 90 |
| clofibric acid + 3-methyl-TTNEB | 425 |

A similar synergistic effect was observed with RXR and RXR-specific ligands and the Vitamin D receptor (VDR) and its cognate ligands. When RXR$\beta$ and VD receptors were co-transfected into CV-1 cells containing a hormone response element, the addition of RXR selective 3-methyl-TTNCB and 1,25-dihydroxy-vitamin D (1,25-D) produced a greater than additive response than was observed for each of the individual ligands, as shown below in Table 6.

TABLE 6

| Compound | Normalized Response (%) |
|---|---|
| 1,25-D | 100 |
| 3-Methyl-TTNCB | 13 |
| 1,25-D + 3-methyl-TTNCB | 190 |

As shown, the above results indicate that each pair of receptors (RXR$\alpha$/PPAR and RXR$\beta$/VDR, respectively), in the presence of ligands known to specifically activate their respective receptors, are capable of producing a synergistic response. The results indicate that the response of a single agent can be enhanced by the combination of the two agents, or that comparable biological or therapeutic responses can be achieved by use of lower doses of such agents in combination.

The observation that RXR-opecific ligands are able to act synergistically with RAR ligands, PPAR ligands, and Vitamin D ligands indicates that RXR-specific ligands have usefulness not only as single therapeutic agents but also in combination therapy to obtain enhanced biological or therapeutic response by the addition of the RXR-specific ligand. Such combination therapy also may provide an added benefit of decreasing the side effects associated with the primary agent by employing lower doses of that agent. For example, use of Vitamin D or a related Vitamin D receptor ligand in conjunction with an RXR selective compound for the treatment of a variety of disorders including skin diseases (acne, psoriasis), hyperproliferative disorders (benign and malignant cancers) and disorders of calcium homeostasis may decrease the adverse side effects associated with Vitamin D therapy alone.

Since RXR is known to form heterodimers with various members of the intracellular receptor super family, it can be expected that the synergistic response observed with use of RXR-selective ligands may be achieved with other receptors with which heterodimers are formed. These include PPARs, RARs, Vitamin D, thyroid hormone receptors, HNF4, the COUP family of receptors, as referenced above, and other as yet unidentified members of the intracellular super family of receptors.

As will be further discernible to those skilled in the art, the compounds disclosed above can be readily utilized in pharmacological applications where selective retinoid receptor activity is desired, and where it is desired to minimize cross reactivities with other related intracellular receptors. *In vivo* applications of the invention include administration of the disclosed compounds to mammalian subjects, and in particular to humans.

The compounds of the present invention are small molecules which are relatively fat soluble or lipophilic and enter the cell by passive diffusion across the plasma membrane. Consequently, these ligands are well suited for administration orally and by injection, as well as topically. Upon administration, these ligands can selectively activate retinoid X receptors, and thereby selectively modulate processes mediated by these receptors.

The pharmaceutical compositions of this invention are prepared in conventional dosage unit forms by incorporating an active compound of the invention, or a mixture of such compounds, with a nontoxic pharmaceutical carrier according to accepted procedures in a nontoxic amount sufficient to produce the desired pharmacodynamic activity in a mammalian and in particular a human subject. Preferably, the composition contains the active ingredient in an active, but nontoxic, amount selected from about 5 mg to about 500 mg of active ingredient per dosage unit. This quantity depends on the specific biological activity desired and the condition of the patient.

The pharmaceutical carrier or vehicle employed may be, for example, a solid or liquid. A variety of pharmaceutical forms can be employed. Thus, when using a solid carrier, the preparation can be plain milled, micronized in oil, tableted, placed in a hard gelatin or enteric-coated capsule in micronized powder or pellet form, or in the form of a troche, lozenge, or suppository. When using a liquid carrier, the preparation can be in the form of a liquid, such as an ampule, or as an aqueous or nonaqueous liquid suspension. For topical administration, the active ingredient may be formulated using bland, moisturizing bases, such as ointments or creams. Examples of suitable ointment bases are petrolatum, petrolatum plus volatile silicones, lanolin, and water in oil emulsions such as Eucerin (Beiersdorf). Examples of suitable cream bases are Nivea Cream (Beiersdorf), cold cream (USP), Purpose Cream (Johnson & Johnson) hydrophilic ointment (USP), and Lubriderm (Warner-Lambert).

The following examples provide illustrative pharmacological composition formulations:

<u>Illustrative Example 4</u>

Hard gelatin capsules are prepared using the following ingredients:

| | Quantity |
|---|---|
| (mg/capsule) | |
| 3-methyl-TTNCB | 140 |
| Starch, dried | 100 |
| Magnesium stearate | 10 |
| Total | 250 mg |

The above ingredients are mixed and filled into hard gelatin capsules in 250 mg quantities.

<u>Illustrative Example 5</u>

A tablet is prepared using the ingredients below:

| | Quantity |
|---|---|
| (mg/tablet) | |
| 3-methyl-TTNCB | 140 |
| Cellulose, microcrystalline | 200 |
| Silicon dioxide, fumed | 10 |
| Stearic acid | 10 |
| Total | 360 mg |

The components are blended and compressed to form tablets each weighing 360 mg.

Illustrative Example 6

Tablets, each containing 60 mg of active ingredient, are made as follows:

| | Quantity |
|---|---|
| (mg/tablet) | |
| 3-methyl-TTNCB | 60 |
| Starch | 45 |
| Cellulose, microcrystalline Polyvinylpyrrolidone (PVP) | 35 |
| (as 10% solution in water) | 4 |
| Sodium carboxymethyl starch (SCMS) | 4.5 |
| Magnesium stearate | 0.5 |
| Talc | 1.0 |
| Total | 150 mg |

The active ingredient, starch, and cellulose are passed through a No. 45 mesh U.S. sieve and mixed thoroughly. The solution of PVP is mixed with the resultant powders, which are then passed through a No. 14 mesh U.S. sieve. The granules so produced are dried at 50°C and passed through a No. 18 mesh U.S. sieve. The SCMS, magnesium stearate, and talc, previously passed through a No. 60 mesh U.S. sieve, are then added to the granules which, after mixing, are compressed on a tablet machine to yield tablets each weighing 150 mg.

Illustrative Example 7

suppositories, each containing 225 mg of active ingredient, may be made as follows:

| | |
|---|---|
| 3-methyl-TTNC | 225 mg |
| Saturated fatty acid glycerides | 2,000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of normal 2g capacity and allowed to cool.

Illustrative Example 8

An intravenous formulation may be prepared as follows:

| | |
|---|---|
| 3-methyl-TTNCB | 100 mg |
| Isotonic saline | 1,000 ml |
| Glycerol | 100 ml |

The compound is dissolved in the glycerol and then the solution is slowly diluted with isotonic saline. The

solution of the above ingredients is then administered intravenously at a rate of 1 ml per minute to a patient.

The compounds of this invention also have utility when labeled as ligands for use in assays to determine the presence of RXRs. They are particularly useful due to their ability to selectively bond to members of the RXR subfamily and can therefore be used to determine the presence of RXR isoforms in the presence of other related receptors.

Due to the selective specificity of the compounds of this invention for retinoid X receptors, these compounds can also be used to purify samples of retinoid X receptors *in vitro*. Such purification can be carried out by mixing samples containing retinoid X receptors with one of more of the bicyclic derivative compounds disclosed so that the compound (ligand) binds to the receptor, and then separating out the bound ligand/receptor combination by separation techniques which are known to those of skill in the art. These techniques include column separation, filtration, centrifugation, tagging and physical separation, and antibody complexing, among others.

## Claims

1. A compound having the formula:

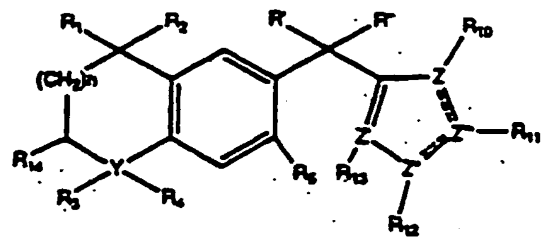

wherein

$R_1$ and $R_2$, each independently, represent hydrogen or lower alkyl or acyl having 1-4 carbon atoms;
Y represents C, O, S, N, CHOH, CO, SO, $SO_2$, or a pharmaceutically acceptable salt;
$R_3$ represents hydrogen or lower alkyl having 1-4 carbon atoms where Y is C or N;
$R_4$ represents hydrogen or lower alkyl having 1-4 carbon atoms where Y is C, but $R_4$ does not exist if Y is N, and neither $R_3$ or $R_4$ exist if Y is S, O, CHOH, CO, SO, or $SO_2$;
R' and R" represent hydrogen, lower alkyl or acyl having 1-4 carbon atoms, OH, alkoxy having 1-4 carbon atoms, thiol or thio ether, or amino,
or R' and R" taken together form an oxo (keto), methano, thioketo, HO-N=, NC-N=, $(R_7R_8)$N-N=, epoxy, cyclopropyl, or cycloalkyl group and wherein the epoxy, cyclopropyl, and cycloalkyl groups can be substituted with lower alkyl having 1-4 carbons or halogen;
$R_5$ represents hydrogen, a lower alkyl having 1-4 carbons, halogen, nitro, $OR_7$, $SR_7$, $NR_7R_8$, or $(CF_2)_nCF_3$
$R_{10}$, $R_{11}$, $R_{12}$ and $R_{13}$ each independently represent hydrogen, a lower alkyl having 1-4 carbons, halogen, nitro, $OR_7$, $SR_7$, $NR_7R_8$ or $(CF_2)_nCF_3$, if the Z, Z', Z" or Z"' from which it originates is C, or each independently represent hydrogen or a lower alkyl having 1-4 carbons if the Z, Z', Z" or Z"' from which it originates is N, with the proviso that one of $R_6$, $R_{10}$, $R_{11}$, $R_{12}$ and $R_{13}$ is X;
each of $R_7$ and $R_8$ independently represents hydrogen or a lower alkyl having 1-6 carbons;
$R_{14}$ represents hydrogen, a lower alkyl having 1-4 carbons, oxo, hydroxy, acyl having 1-4 carbons, halogen, thiol or thioketone; .
X is COOH, tetrazole, $PO_3H$, $SO_3H$, CHO, $CH_2OH$, $CONH_2$, COSH, $COOR_9$, $COSR_9$, $CONHR_9$, or COOW where $R_9$ represents a lower alkyl having 1-4 carbons, phenyl, aromatic alkyl, or q-hydroxyphenyl, q-bromophenyl, q-chlorophenyl, q-fluorophenyl, or q-iodophenyl, where q=2-4, where W is a pharmaceutically acceptable salt;
one of Z, Z', Z" and Z"' represents S and the rest are C;
n=0-3; and
the dashed lines depict optional double bonds.

2. A compound of claim 1 wherein said compound selectively activates Retinoid X Receptors in preference to Retinoic Acid Receptors.

3. A compound according to claim 1, of any of the following formulae:

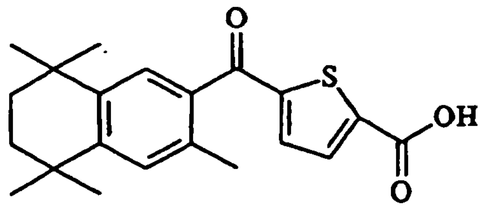

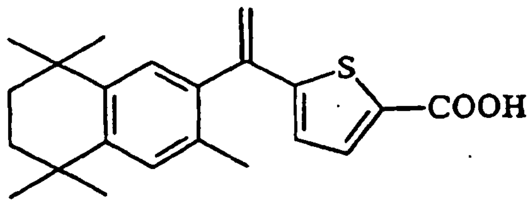

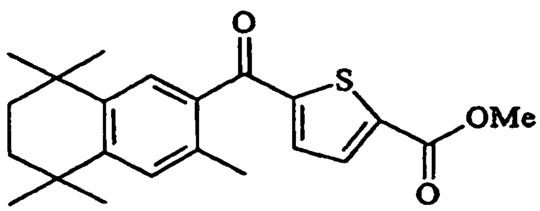

4. A pharmaceutical composition comprising in a pharmaceutically acceptable vehicle suitable for enteral, parenteral, or topical administration, one or more compound of claim 1 or 2 or 3.

5. A method for modulating a process of in vitro cellular growth and differentiation mediated by one or more Retinoid X Receptors, said method comprising causing said process to be conducted in the presence of at least one compound of any of claims 1 to 3.

6. A method according to claim 5 wherein said Retinoid X Receptor is Retinoid X Receptor-alpha, Retinoid X Receptor-beta, or Retinoid X Receptor-gamma.

7. An in vitro method for modulating a process mediated by one or more Retinoid X Receptors, said method comprising causing said process to be conducted in the presence of at least one compound as set forth in claim 3.

8. A method for determining the presence of one or more Retinoid X Receptors comprising combining a compound according to any of claims 1 to 3 with a sample containing one or more unknown receptors and determining whether said ligand binds to any receptor in said sample.

9. A method of purifying Retinoid X Receptors comprising combining a compound according to any of claims 1 to 3 with a sample containing one or more said Retinoid X Receptors, allowing said compound to bind with Retinoid X Receptors, and separating out the bound combination of said compound and Retinoid X Receptor.

10. A composition comprising a first ligand which selectively activates Retinoid X Receptors in preference to Retinoic Acid Receptors, in combination with a second ligand which selectively activates Retinoic Acid Receptors in preference to Retinoid X Receptors ; the first ligand being a compound according to any of claims 1 to 3.

11. A composition comprising a first ligand which selectively activates Retinoid X Receptors in preference to Retinoic Acid Receptors, in combination with a second ligand which activates one or more intracellular receptors other than

Retinoid X Receptors ; the first ligand being a compound according to any of claims 1 to 3.

**12.** The composition of claim 10 or 11 wherein the physiological effect in mammals produced by said composition at a given concentration is greater than the additive effect achieved utilizing each said ligand alone at said concentration.

**13.** A pharmaceutical composition comprising in a pharmaceutically acceptable vehicle for enteral, parenteral, or topical administration a first ligand which selectively activates Retinoid X Receptors in preference to Retinoic Acid Receptors, in combination with a second ligand which selectively activates one or more intracellular receptors other than Retinoid X Receptors ; the first ligand being a compound according to any of claims 1 to 3.

**14.** A pharmaceutical composition of claim 13 wherein said second ligand selectively activates Retinoic Acid Receptors in preference to Retinoid X Receptors.

**15.** An in vitro method for modulating a process mediated by intracellular receptors, said method comprising causing said process to be conducted in the presence of a composition comprising a first ligand which selectively activates Retinoid X Receptors in preference to Retinoic Acid Receptors, in combination with a second ligand which activates one or more intracellular receptors other than Retinoid X Receptors, and wherein the physiological effect in mammals produced by said composition at a given concentration is greater than the additive effect achieved utilizing each said ligand alone at said concentration; the first ligand being a compound according to any of claims 1 to 3.

**16.** The method of claim 15 wherein said composition is present at a concentration at which neither said first nor second ligand would alone produce a significant therapeutic response.

**17.** The method of claim 15 wherein said second ligand activates peroxisome proliferator activated receptors.

**18.** The method of claim 15 wherein said second ligand activates Vitamin D receptors.

**19.** The method of claim 15 wherein said second ligand activates thyroid hormone receptors, HNF4 receptors, or members of the COUP family of receptors.

**20.** Use of a compound according to any of claims 1 to 3 in the preparation of a medicament for the modulation of in vivo lipid metabolism, skin-related processes, programmed cell death, malignant cell development, premalignant lesions, autoimmune diseases or fatty acid metabolism.

**21.** A compound according to any of claims 1 to 3 for administering to a mammalian subject to modulate a process mediated by one or more Retinoid X Receptors.

**Patentansprüche**

**1.** Verbindung mit der Formel:

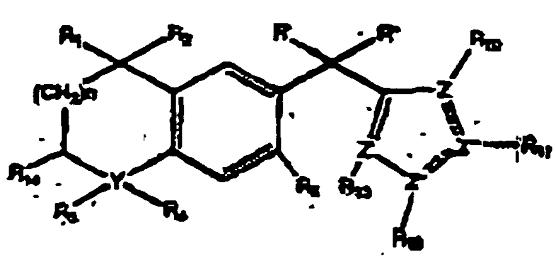

worin

$R_1$ und $R_2$ jeweils unabhängig für Wasserstoff oder niederes Alkyl oder Acyl mit 1-4 Kohlenstoffatomen stehen;
Y für C, O, S, N, CHOH, CO, SO, $SO_2$ oder ein pharmazeutisch akzeptables Salz steht;

$R_3$ für Wasserstoff oder niederes Alkyl mit 1-4 Kohlenstoffatomen steht, worin Y C oder N ist;
$R_4$ für Wasserstoff oder niederes Alkyl mit 1-4 Kohlenstoffatomen steht, worin Y C ist, doch $R_4$ nicht vorhanden ist, wenn Y N ist, und weder $R_3$ noch $R_4$ vorhanden sind, wenn Y S, O, CHOH, CO, SO oder $SO_2$ ist;
R' und R" für Wasserstoff, niederes Alkyl oder Acyl mit 1-4 Kohlenstoffatomen, OH, Alkoxy mit 1-4 Kohlenstoffatomen, Thiol oder Thioethler, oder Amino stehen; oder R' und R" zusammen genommen eine Oxo (Keto), Methano, Thioketo, HO-N=, $(R_7R_8)$N-N=, Epoxy-, Cyclopropyl- oder Cycloalkylgruppe bilden und worin die Epoxy-, Cyclopropyl- und Cycloalkylgruppen mit niederem Alkyl mit 1-4 Kohlenstoffen oder Halogen substituiert werden können;
$R_5$ für Wasserstoff, ein niederes Alkyl mit 1-4 Kohlenstoffen, Halogen, Nitro, $OR_7$, $SR_7$, $NR_7R_8$ oder $(CF_2)_nCF_3$ steht;
$R_{10}$, $R_{11}$, $R_{12}$ und $R_{13}$ jeweils unabhängig für Wasserstoff, ein niederes Alkyl mit 1-4 Kohlenstoffen, Halogen, Nitro, $OR_7$, $SR_7$, $NR_7R_8$ oder $(CF_2)_nCF_3$ stehen, wenn das Z, Z', Z" oder Z"', von dem es entspringt, C ist, oder jeweils unabhängig für Wasserstoff oder ein niederes Alkyl mit 1-4 Kohlenstoffen stehen, wenn das Z, Z', Z" oder Z"', von dem es entspringt, N ist, unter der Voraussetzung, dass eines von $R_6$, $R_{10}$, $R_{11}$, $R_{12}$ und $R_{13}$ X ist;
jedes von $R_7$ und $R_8$ unabhängig für Wasserstoff oder ein niederes Alkyl mit 1-6 Kohlenstoffen steht;
$R_{14}$ für Wasserstoff, ein niederes Alkyl mit 1-4 Kohlenstoffen, Oxo, Hydroxy, Acyl mit 1-4 Kohlenstoffen, Halogen, Thiol oder Thioketon steht;
X COOH, Tetrazol, $PO_3H$, $SO_3H$, CHO, $CH_2OH$, $CONH_2$, COSH, $COOR_9$, $COSR_9$, $CONHR_9$ oder COOW ist, worin $R_9$ für ein niederes Alkyl mit 1-4 Kohlenstoffen, Phenyl, aromatisches Alkyl oder q-Hydroxyphenyl, q-Bromphenyl, q-Chlorphenyl, q-Fluorphenyl oder q-Iodphenyl steht, worin q=2-4, wobei W ein pharmazeutisch akzeptables Salz ist;
eines von Z, Z', Z" oder Z"' für S steht und der Rest C sind;
n=0-3; und
die gestrichelten Linien optionale Doppelbindungen darstellen.

**2.** Verbindung nach Anspruch 1, wobei die Verbindung selektiv Retinoid-X Rezeptoren bevorzugt vor Retinsäure-Rezeptoren aktiviert.

**3.** Verbindung nach Anspruch 1, von einer der folgenden Formeln:

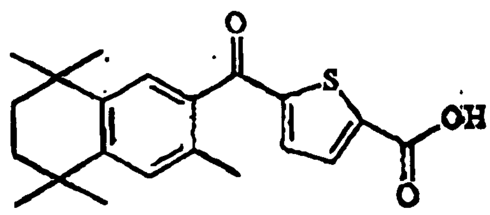

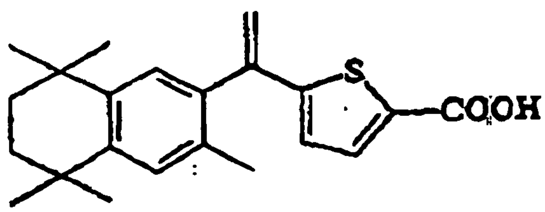

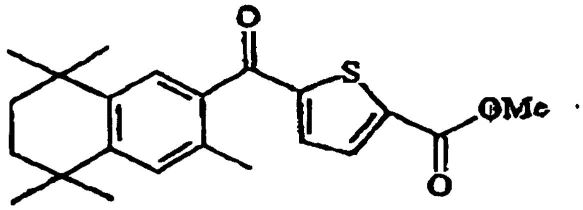

4. Pharmazeutische Zusammensetzung, umfassend in einem pharmazeutisch akzeptablen Vehikel, das für die enterale, parenterale oder topische Verabreichung geeignet ist, ein oder mehrere Verbindungen nach Anspruch 1 oder 2 oder 3.

5. Methode zum Modulieren eines Vorgangs der In vitro-Zellkultivierung und -differenzierung, wie durch ein oder mehrere Retinoid-X-Rezeptoren vermittelt, welche Methode das Bewirken des Ablaufs des Vorgangs in Gegenwart mindestens einer Verbindung nach einem der Ansprüche 1 bis 3 umfasst.

6. Methode nach Anspruch 5, wobei der Retinoid-X-Rezeptor Retinoid-X-Rezeptoralpha, Retinoid-X-Rezeptor-beta oder Retinoid-X-Rezeptor-gamma ist.

7. In-vitro-Methode zum Modulieren eines Vorgangs, wie durch ein oder mehrere Retinoid-X-Rezeptoren vermittelt, welche Methode das Bewirken des Ablaufs des Vorgangs in Gegenwart mindestens einer Verbindung nach Anspruch 3 umfasst.

8. Methode zum Bestimmen des Vorhandenseins eines oder mehrerer Retinoid-X-Rezeptoren, umfassend das Kombinieren einer Verbindung nach einem der Ansprüche 1 bis 3 mit einer Probe, die ein oder mehrere unbekannte Rezeptoren enthält, und das Bestimmen, ob der Ligand an irgendeinen Rezeptor in der Probe bindet.

9. Methode zum Reinigen von Retinoid-X-Rezeptoren, umfassend das Kombinieren einer Verbindung nach einem der Ansprüche 1 bis 3 mit einer Probe, die ein oder mehrere der Retinoid-X-Rezeptoren enthält, das Bindenlassen der Verbindung mit Retinoid-X-Rezeptoren und das Heraustrennen der gebundenen Kombination aus der Verbindung und Retinoid-X-Rezeptor.

10. Zusammensetzung, umfassend einen ersten Liganden, welcher selektiv Retinoid-X-Rezeptoren bevorzugt vor Retinsäure-Rezeptoren aktiviert, in Kombination mit einem zweiten Liganden, welcher selektiv Retinsäure-Kezeptoren bevorzugt vor Retinoid-X-Rezeptoren aktiviert, wobei der erste Ligand eine Verbindung nach einem der Ansprüche 1 bis 3 ist.

11. Zusammensetzung, umfassend einen ersten Liganden, welcher selektiv Retinoid-X-Rezeptoren bevorzugt vor Retinsäure-Rezeptoren aktiviert, in Kombination mit einem zweiten Liganden, welcher ein oder mehrere intrazelluläre Rezeptoren außer Retinoid-X-Rezeptoren aktiviert, wobei der erste Ligand eine Verbindung nach einem der Ansprüche 1 bis 3 ist.

12. Zusammensetzung nach Anspruch 10 oder 11, wobei die physiologische Wirkung bei Säugern, wie durch die Zusammensetzung bei einer gegebenen Konzentra tion erzeugt, größer ist als die additive Wirkung, die unter Verwendung jedes der Liganden alleine bei dieser Konzentration erzielt wird.

13. Pharmazeutische Zusammensetzung, umfassend in einem pharmazeutisch akzeptablen Vehikel für die enterale, parenterale oder topische Verabreichung einen ersten Liganden, welcher selektiv Retinoid-X-Rezeptor bevorzugt vor Retinsäure-Rezeptoren aktiviert, in Kombination mit einem zweiten Liganden, welcher selektiv ein oder mehrere intrazelluläre Rezeptoren außer Retinoid-X-Rezeptoren aktiviert, wobei der erste Ligand eine Verbindung nach einem der Ansprüche 1 bis 3 ist.

14. Pharmazeutische Zusammensetzung nach Anspruch 13, wobei der zweite Ligand selektiv Retinsäure-Rezeptoren bevorzugt vor Retinoid-X-Rezeptoren aktiviert.

15. In-vitro-Methode zum Modulieren eines Vorgangs, wie durch intrazelluläre Rezeptoren vermittelt, welche Methode das Bewirken des Ablaufs des Vorgangs in Gegenwart einer Verbindung umfasst, die einen ersten Liganden umfasst, welcher selektiv Retinoid-X-Rezeptor bevorzugt vor Retinsäure-Rezeptoren aktiviert, in Kombination mit einem zweiten Liganden, welcher ein oder mehrere intrazelluläre Rezeptoren außer Retinoid-X-Rezeptoren aktiviert, und wobei die physiologische Wirkung bei Säugem, wie durch die Zusammensetzung bei einer gegebenen Konzentration erzeugt, größer ist als die additive Wirkung, die unter Verwendung jedes der Liganden alleine bei dieser Konzentration erzielt wird; wobei der erste Ligand eine Verbindung nach einem der Ansprüche 1 bis 3 ist.

**16.** Methode nach Anspruch 16, wobei die Zusammensetzung bei einer Konzentration vorhanden ist, bei der weder der erste noch der zweite Ligand alleine eine signifikante therapeutische Reaktion erzeugen würde.

**17.** Methode nach Anspruch 15, wobei der zweite Ligand Peroxisom-Proliferatoraktivierte Rezeptoren aktiviert.

**18.** Methode nach Anspruch 15, wobei der zweite Ligand Vitamin-D-Rezeptoren aktiviert.

**19.** Methode nach Anspruch 15, wobei der zweite Ligand Schilddrüsenhormon-Rezeptoren, $HNF_4$-Rezeptoren oder Mitglieder der COUP-Familie der Rezeptoren aktiviert.

**20.** Verwendung einer Verbindung nach einem der Ansprüche 1 bis 3 in der Herstellung eines Medikaments für die Modulation des in vivo-Lipidstoffwechsels, hautbezogener Prozesse, des programmierten Zelltods, der malignen Zellentwicklung, prämaligner Läsionen, Autoimmunerkrankungen oder des Fettsäurestoffwechsels.

**21.** Verbindung nach einem der Ansprüche 1 bis 3 zur Verabreichung an einen Säuger, zur Modulation eines Vorgangs, der durch ein oder mehrere Retinoid-X-Rezeptoren vermittelt ist.

## Revendications

**1.** Composé ayant la formule :

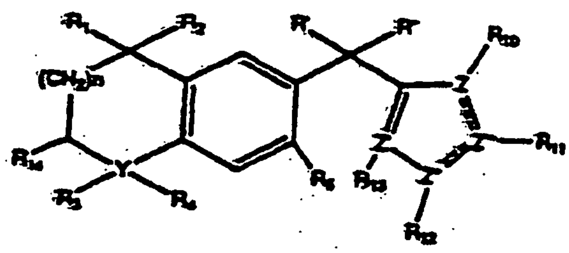

dans lequel

$R_1$ et $R_2$, chacun indépendamment, représentent un hydrogène ou un alkyle ou acyle inférieur ayant de 1 à 4 atomes de carbone ;
Y représente C, O, S, N, CHOH, CO, SO, $SO_2$, ou un sel pharmaceutiquement acceptable ;
$R_3$ représente un hydrogène ou un alkyle inférieur ayant de 1 à 4 atomes de carbone où Y est C ou N ;
$R_4$ représente un hydrogène ou un alkyle inférieur ayant de 1 à 4 atomes de carbone lorsque Y est C, mais $R_4$ n'existe pas si Y est N, et ni $R_3$, ni $R_4$ n'existent si Y est S, O, CHOH, CO, SO, ou $SO_2$ ;
R' et R" représentent un hydrogène, un alkyle ou acyle inférieur ayant de 1 à 4 atomes de carbones, OH, un alcoxy ayant de 1 à 4 atomes de carbone, un thiol ou un thioéther, ou un amino,
ou R' et R" forment conjointement un groupe oxo (céto), méthano, thiocéto, HO-N=, NC-N=, $(R_7R_8)N$-N=, époxy, cyclopropyle ou cycloalkyle et dans lequel les groupes époxy, cyclopropyle et cycloalkyle peuvent être substitués avec un alkyle inférieur ayant de 1 à 4 atomes de carbone ou un halogène ;
$R_5$ représente un hydrogène, un alkyle inférieur ayant de 1 à 4 carbones, un halogène, un nitro, $OR_7$, $SR_7$, $NR_7R_8$, ou $(CF_2)_nCF_3$.
$R_{10}$, $R_{11}$, $R_{12}$ et $R_{13}$ représentent chacun indépendamment un hydrogène, un alkyle inférieur ayant de 1 à 4 carbones, un halogène, un nitro, $OR_7$, $SR_7$, $NR_7R_8$, ou $(CF_2)_nCF_3$, si Z, Z', Z' ou Z''' desquels ils proviennent est C, ou représentent chacun indépendamment un hydrogène ou un alkyle inférieur ayant de 1 à 4 carbones si Z, Z', Z' ou Z''' desquels ils proviennent est N, à condition que l'un parmi $R_6$, $R_{10}$, $R_{11}$, $R_{12}$ et $R_{13}$ est X ;

chacun parmi $R_7$ et $R_8$ représente indépendamment un hydrogène ou un alkyle inférieur ayant de 1 à 6 carbones ;
$R_{14}$ représente un hydrogène, un alkyle inférieur ayant de 1 à 4 carbones, un oxo, un hydroxy, un acyle ayant de 1 à 4 carbones, un halogène, un thiol ou une thiocétone ;
X est COOH, un tétrazole, $PO_3H$, $SO_3H$, CHO, $CH_2OH$, $CONH_2$, COSH, $COOR_9$, $COSR_9$, $CONHR_9$ ou COOW où $R_9$ représente un alkyle inférieur ayant de 1 à 4 carbones, un phényle, un alkyle aromatique, ou un q-hydroxyphényle, un q-bromophényle, un q-chlorophényle, un q-fluorophényle, ou un q-iodophényle, où q = 2 à 4, où W est un sel pharmaceutiquement acceptable ;
l'un parmi Z, Z', Z" et Z"' représente S et le reste est C ;
n = 0 à 3 ; et
les traits tiretés désignent des doubles liaisons facultatives.

**2.** Composé de la revendication 1 dans lequel ledit composé active sélectivement les récepteurs de rétinoïde X de préférence aux récepteurs d'acide rétinoïque.

**3.** Composé selon la revendication 1, de l'une quelconque des formules suivantes :

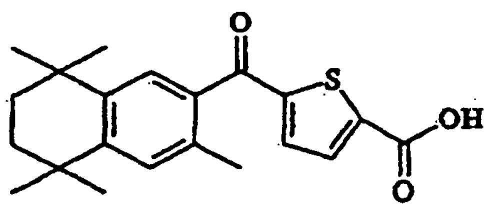

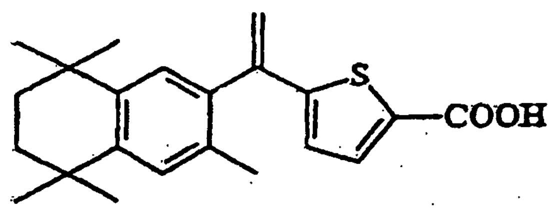

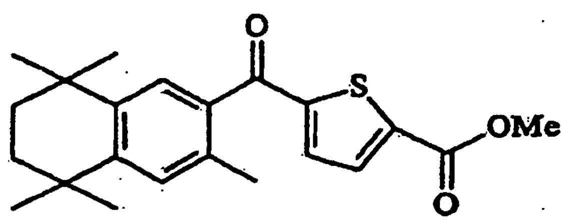

**4.** Composition pharmaceutique comprenant dans un véhicule pharmaceutiquement acceptable adapté pour l'administration entérale, parentérale ou locale, un ou plusieurs composés de la revendication 1 ou 2 ou 3.

**5.** Procédé de modulation d'un processus de croissance et de différenciation cellulaire *in vitro* à médiation par un ou plusieurs récepteurs de rétinoïde X, ledit procédé comprenant la conduite dudit processus en présence d'au moins un composé de l'une quelconque des revendications 1 à 3.

**6.** Procédé selon la revendication 5 dans lequel ledit récepteur de rétinoïde X est le récepteur de rétinoïde X alpha, le récepteur de rétinoïde X bêta ou le récepteur de rétinoïde X gamma.

**7.** Procédé *in vitro* pour moduler un processus à médiation par un ou plusieurs récepteurs de rétinoïde X, ledit procédé

comprenant la conduite dudit processus en présence d'au moins un composé comme décrit dans la revendication 3.

**8.** Procédé pour déterminer la présence d'un ou plusieurs récepteurs de rétinoïde X comprenant la combinaison d'un composé selon l'une quelconque des revendications 1 à 3 avec un échantillon contenant un ou plusieurs récepteurs inconnus et la détermination de la liaison ou non du ligand à un récepteur quelconque dans ledit échantillon.

**9.** Procédé de purification de récepteurs de rétinoïde X comprenant la combinaison d'un composé selon l'une quelconque des revendications 1 à 3 avec un échantillon contenant un ou plusieurs desdits récepteurs de rétinoïde X, le fait de rendre possible la liaison dudit composé aux récepteurs de rétinoïde X, et la séparation de la combinaison liée dudit composé et du récepteur de rétinoïde X.

**10.** Composition comprenant un premier ligand qui active sélectivement les récepteurs de rétinoïde X de préférence aux récepteurs d'acide rétinoïque, en combinaison avec un second ligand qui active sélectivement les récepteurs d'acide rétinoïque de préférence aux récepteurs de rétinoïde X, le premier ligand étant un composé selon l'une quelconque des revendications 1 à 3.

**11.** Composition comprenant un premier ligand qui active sélectivement les récepteurs de rétinoïde X de préférence aux récepteurs d'acide rétinoïque, en combinaison avec un second ligand qui active un ou plusieurs récepteurs intracellulaires autres que des récepteurs de rétinoïde X, le premier ligand étant un composé selon l'une quelconque des revendications 1 à 3.

**12.** Composition de la revendication 10 ou 11 dans laquelle l'effet physiologique chez des mammifères produit par ladite composition à une concentration donnée est supérieur à l'effet additif obtenu en utilisant chacun desdits ligands seul à ladite concentration.

**13.** Composition pharmaceutique comprenant dans un véhicule pharmaceutiquement acceptable pour l'administration entérale, parentérale ou locale, un premier ligand qui active sélectivement les récepteurs de rétinoïde X de préférence aux récepteurs d'acide rétinoïque, en combinaison avec un second ligand qui active sélectivement un ou plusieurs récepteurs intracellulaires autres que des récepteurs de rétinoïde X, le premier ligand étant un composé selon l'une quelconque des revendications 1 à 3.

**14.** Composition pharmaceutique de la revendication 13 dans laquelle ledit second ligand active sélectivement les récepteurs d'acide rétinoïque de préférence aux récepteurs de rétinoïde X.

**15.** Procédé *in vitro* pour moduler un processus à médiation par des récepteurs intracellulaires, ledit procédé comprenant la conduite dudit processus en présence d'une composition comprenant un premier ligand qui active sélectivement les récepteurs de rétinoïde X de préférence aux récepteurs d'acide rétinoïque, en combinaison avec un second ligand qui active un ou plusieurs récepteurs intracellulaires autres que des récepteurs de rétinoïde X, et dans lequel l'effet physiologique chez des mammifères produit par ladite composition à une concentration donnée est supérieur à l'effet additif obtenu en utilisant chacun desdits ligands seul à ladite concentration, le premier ligand étant un composé selon l'une quelconque des revendications 1 à 3.

**16.** Procédé de la revendication 15 dans lequel ladite composition est présente à une concentration à laquelle aucun desdits premier et second ligand ne produirait seul une réponse thérapeutique significative.

**17.** Procédé de la revendication 15 dans lequel ledit second ligand active des récepteurs activés des proliférateurs de peroxysome.

**18.** procédé de la revendication 15 dans lequel ledit second ligand active des récepteurs de vitamine D.

**19.** Procédé de la revendication 15 dans lequel ledit second ligand active des récepteurs d'hormone thyroïdienne, des récepteurs de HNF4, ou des membres de la famille de récepteurs COUP.

**20.** Utilisation d'un composé selon l'une quelconque des revendications 1 à 3 dans la préparation d'un médicament pour la modulation du métabolisme des lipides *in vivo,* de processus associés à la peau, de la mort cellulaire programmée, du développement de cellules malignes, des lésions prémalignes, des maladies auto-immunes ou du métabolisme des acides gras.

**21.** Composé selon l'une quelconque des revendications 1 à 3 pour l'administration à un sujet mammifère pour moduler un processus à médiation par un ou plusieurs récepteurs de rétinoïde X.

FIG. 1.

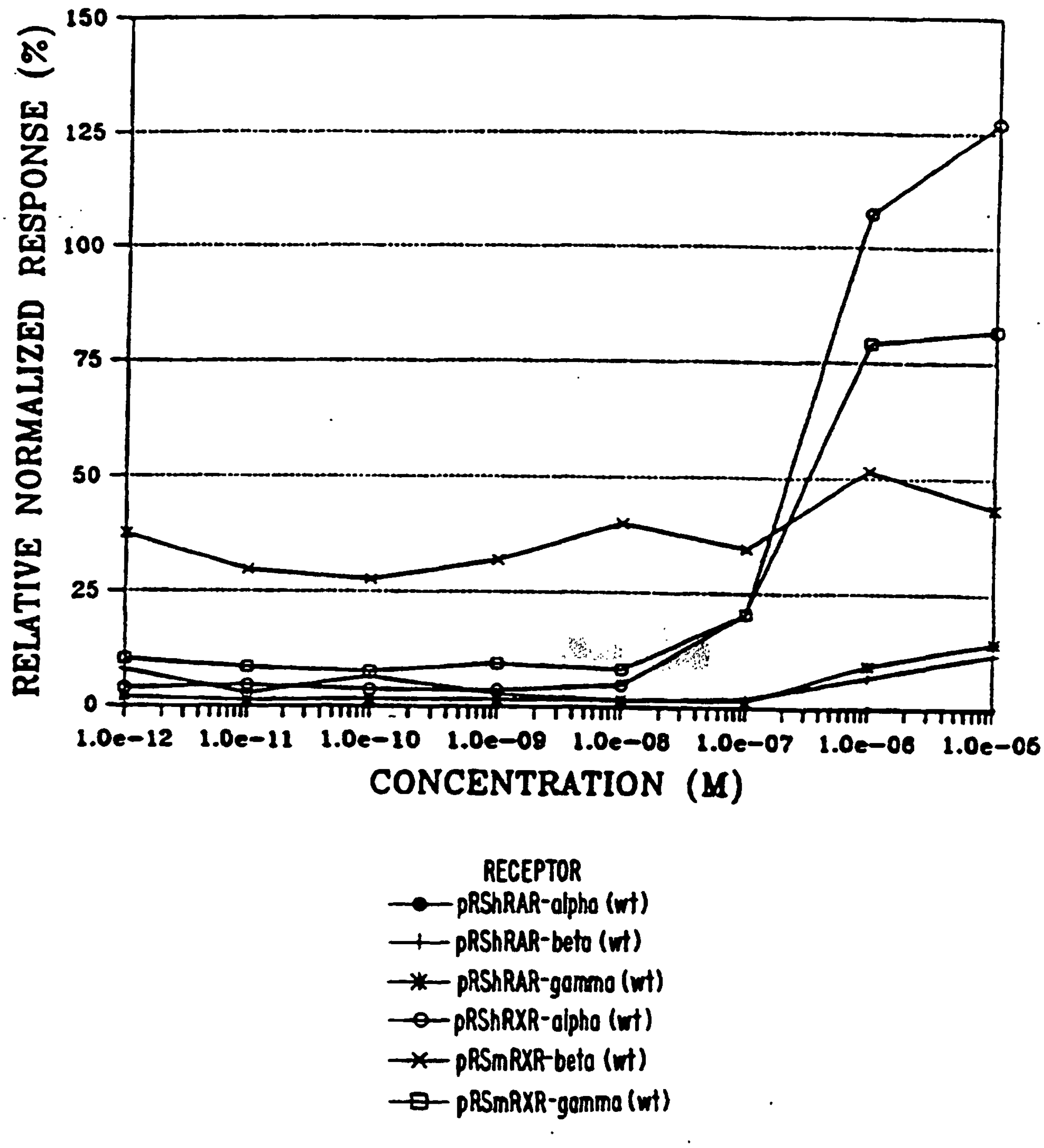

RELATIVE NORMALIZED RESPONSE (%)
150
125
100
75
50
25
0
1.0e-12
1.0e-11
1.0e-10
1.0e-09
1.0e-08
1.0e-07
1.0e-06
1.0e-05
CONCENTRATION (M)
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)

FIG. 2.

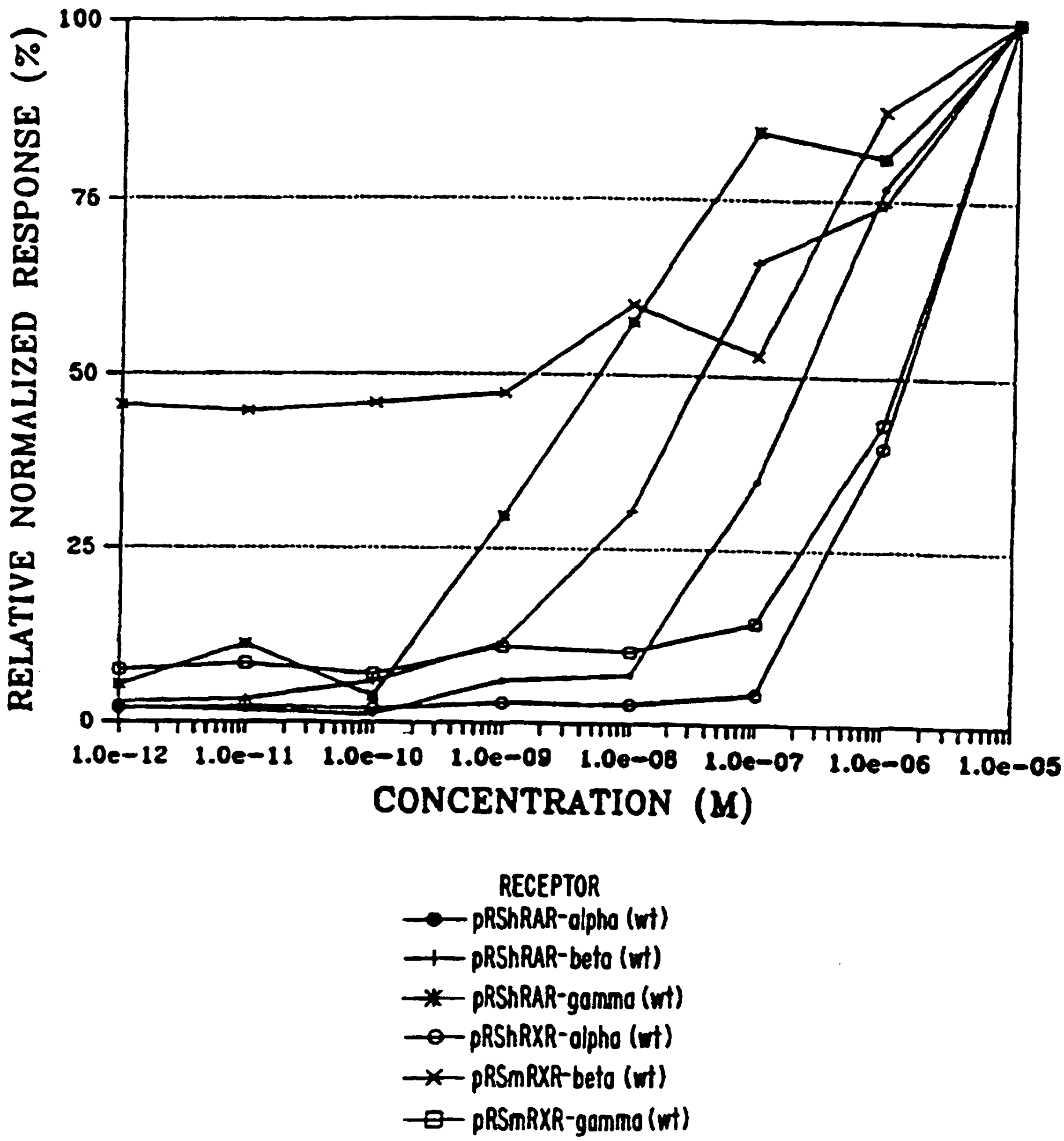
RELATIVE NORMALIZED RESPONSE (%)
100
75
50
25
0
1.0e-12
1.0e-11
1.0e-10
1.0e-09
1.0e-08
1.0e-07
1.0e-06
1.0e-05
CONCENTRATION (M)
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)

FIG. 3

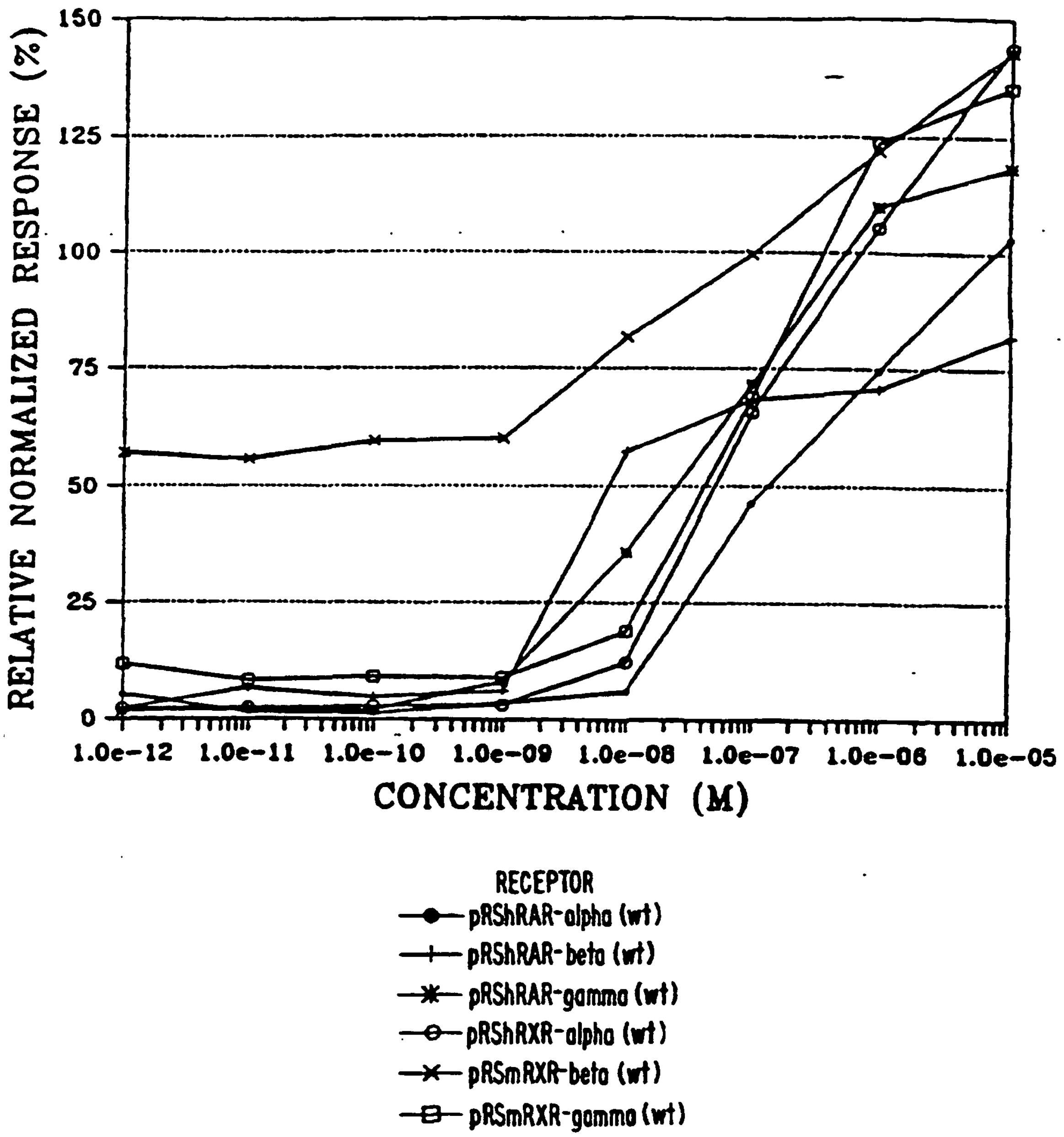
RELATIVE NORMALIZED RESPONSE (%)
150
125
100
75
50
25
0
1.0e-12
1.0e-11
1.0e-10
1.0e-09
1.0e-08
1.0e-07
1.0e-06
1.0e-05
CONCENTRATION (M)
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)

FIG. 4.

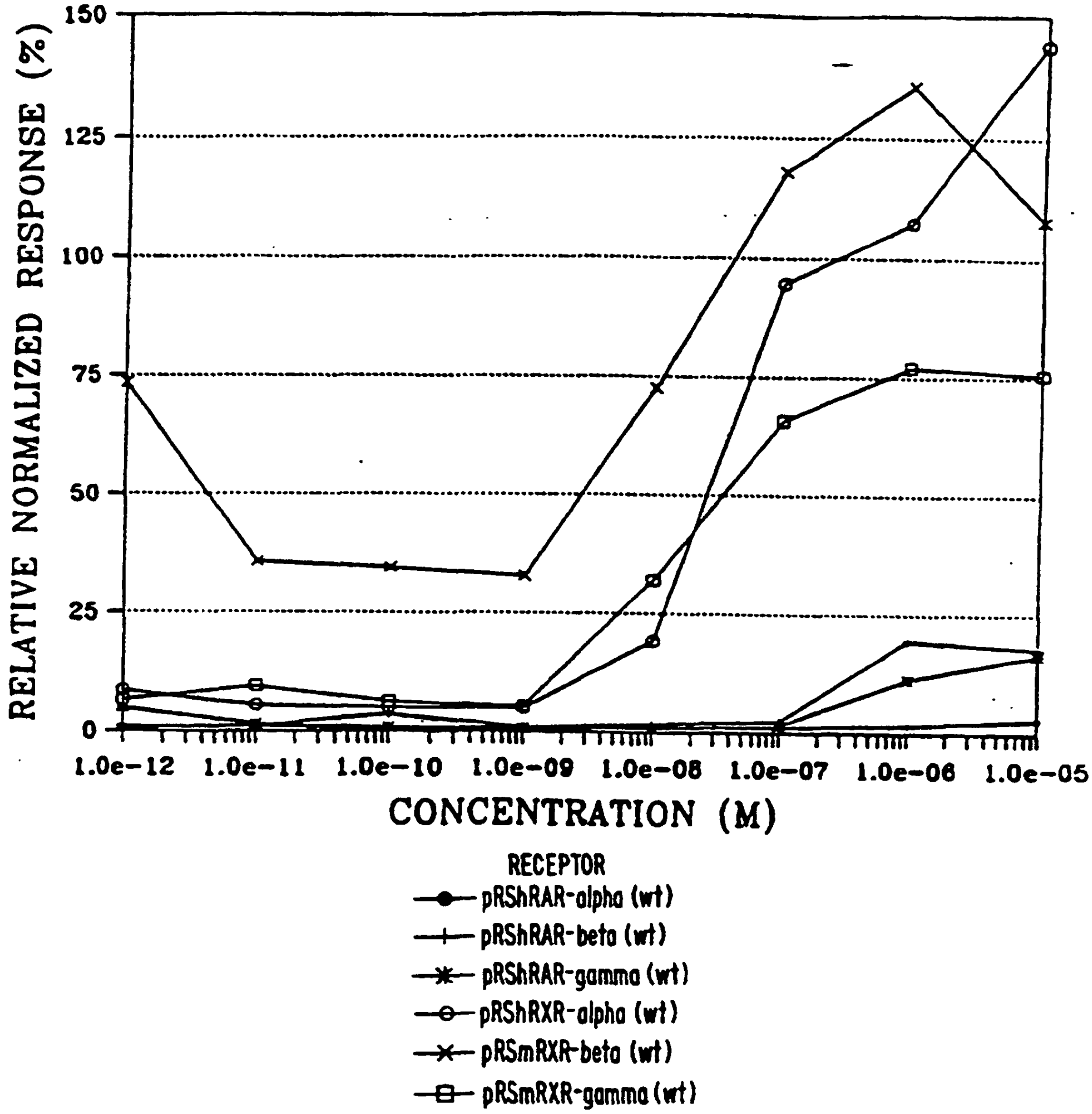
150
125
100
75
50
25
0
RELATIVE NORMALIZED RESPONSE (%)
1.0e-12
1.0e-11
1.0e-10
1.0e-09
1.0e-08
1.0e-07
1.0e-06
1.0e-05
CONCENTRATION (M)
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)

FIG. 5.

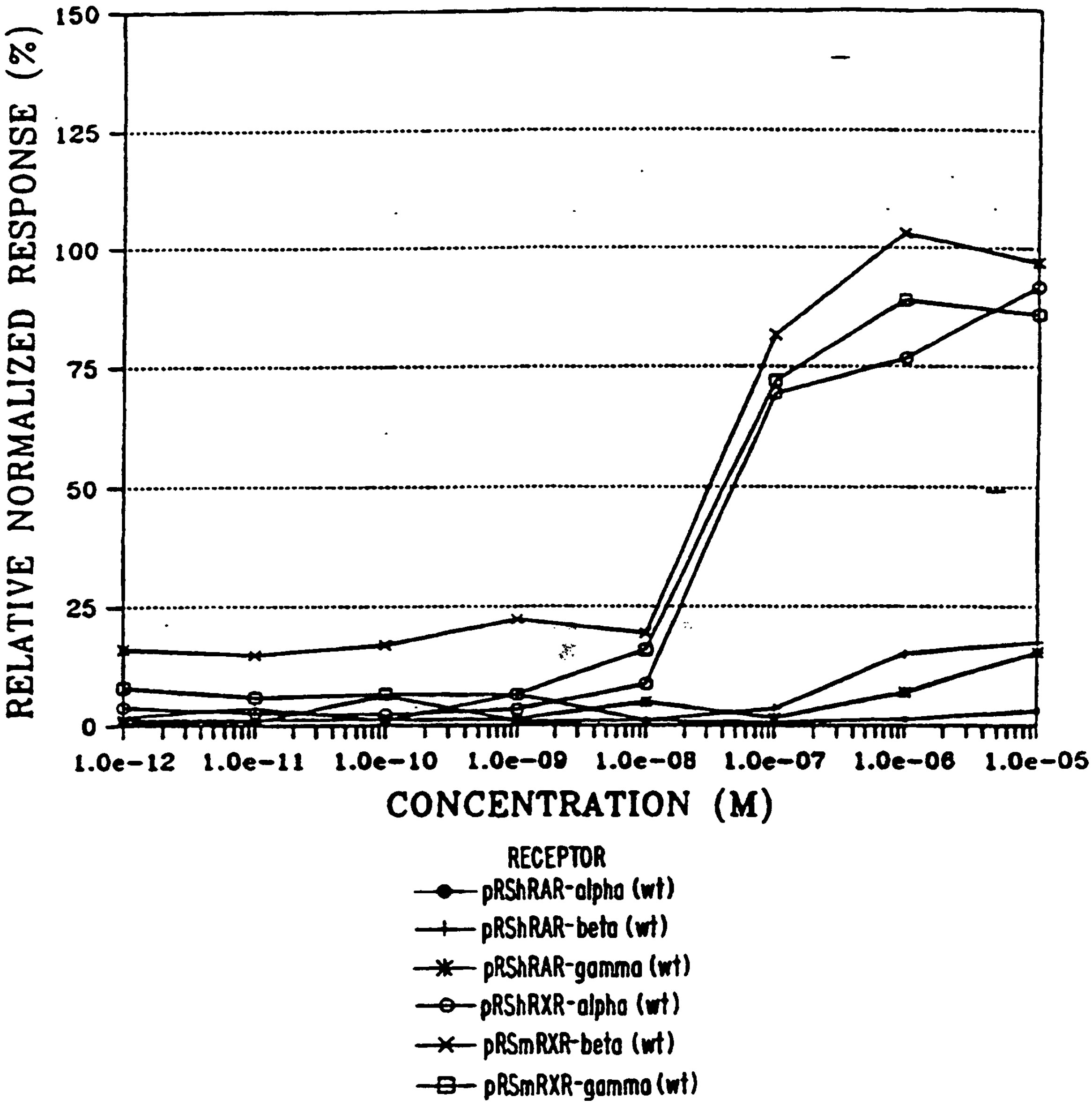
RELATIVE NORMALIZED RESPONSE (%)
150
125
100
75
50
25
0
1.0e-12
1.0e-11
1.0e-10
1.0e-09
1.0e-08
1.0e-07
1.0e-06
1.0e-05
CONCENTRATION (M)
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)

FIG. 6.

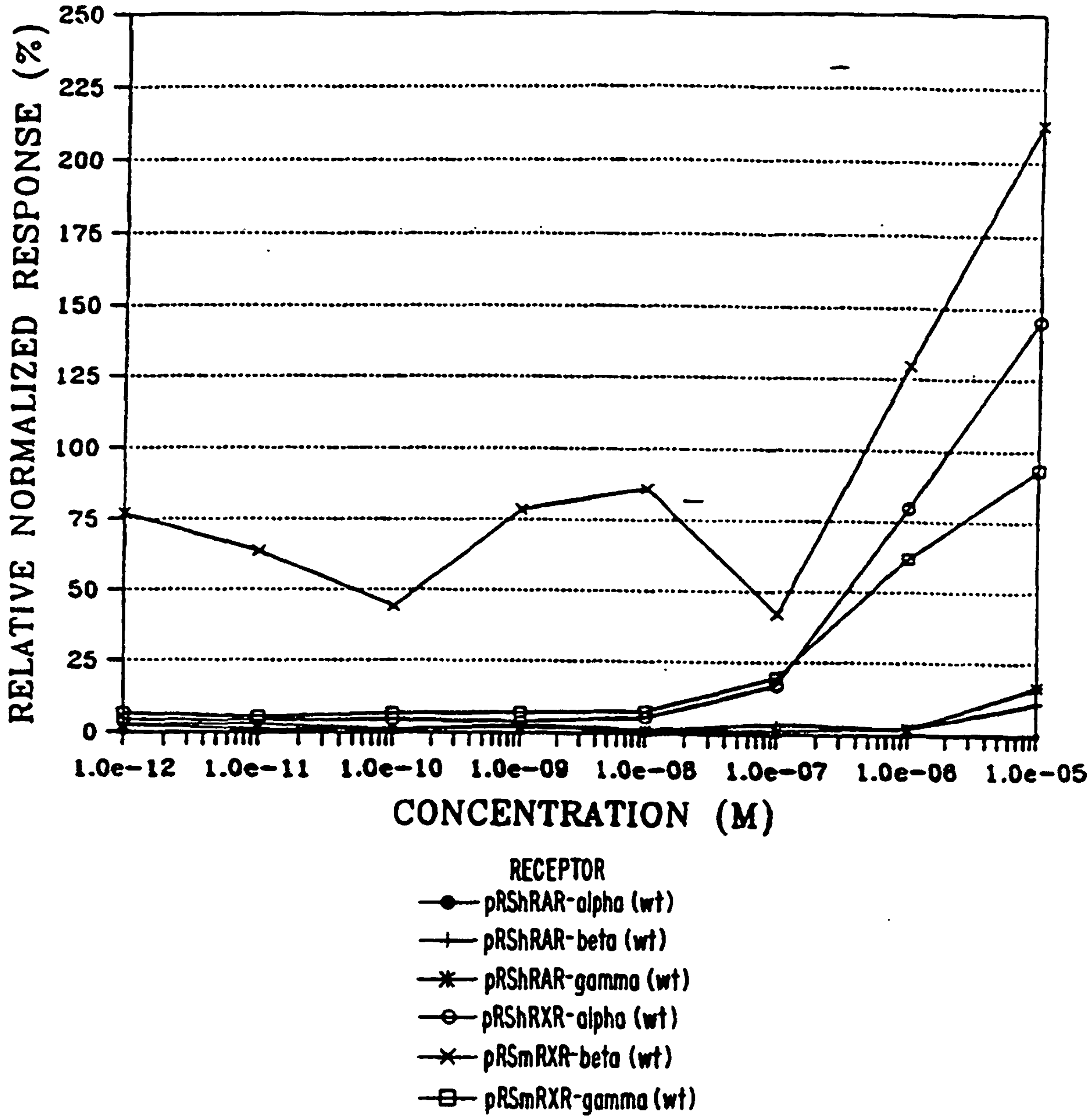
250
225
200
175
150
125
100
75
50
25
0
RELATIVE NORMALIZED RESPONSE (%)
1.0e-12
1.0e-11
1.0e-10
1.0e-09
1.0e-08
1.0e-07
1.0e-06
1.0e-05
CONCENTRATION (M)
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)

FIG. 7.

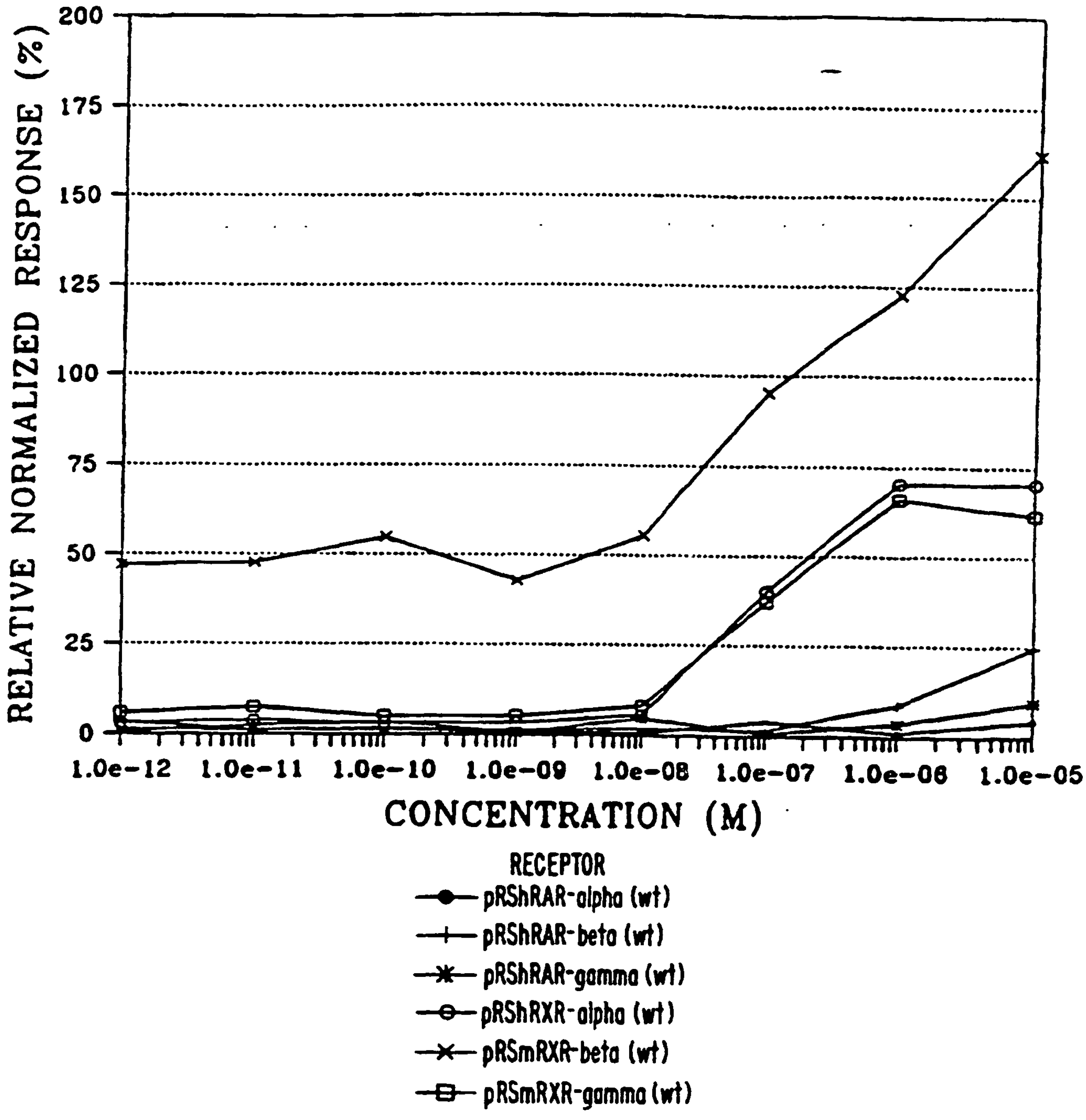
RELATIVE NORMALIZED RESPONSE (%)
200
175
150
125
100
75
50
25
0
1.0e-12
1.0e-11
1.0e-10
1.0e-09
1.0e-08
1.0e-07
1.0e-06
1.0e-05
CONCENTRATION (M)
RECEPTOR
pRShRAR-alpha (wt)
pRShRAR-beta (wt)
pRShRAR-gamma (wt)
pRShRXR-alpha (wt)
pRSmRXR-beta (wt)
pRSmRXR-gamma (wt)

FIG. 8.

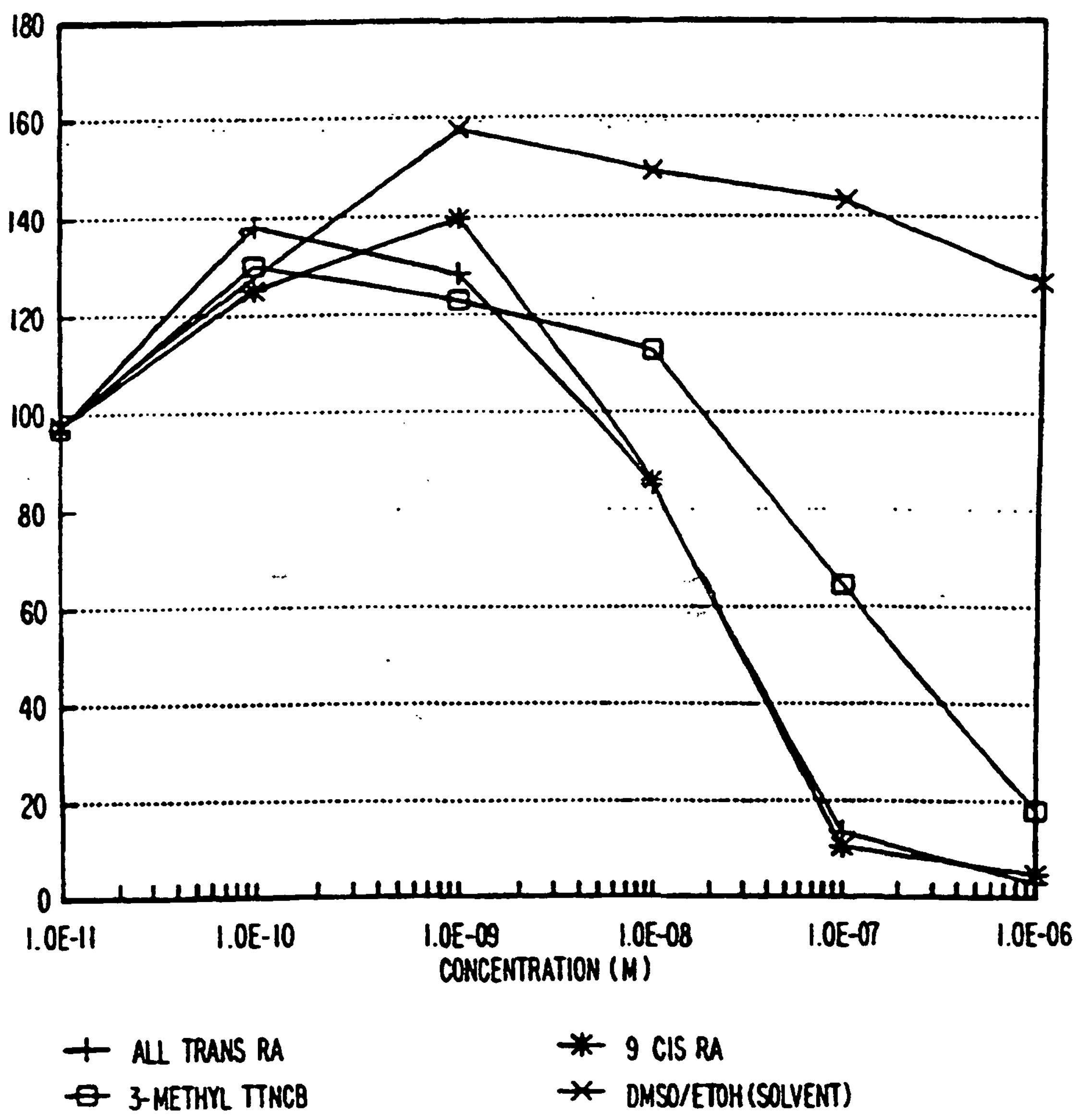
180
160
140
120
100
80
60
40
20
0
1.0E-11
1.0E-10
1.0E-09
1.0E-08
1.0E-07
1.0E-06
CONCENTRATION (M)
ALL TRANS RA
3-METHYL TTNCB
9 CIS RA
DMSO/ETOH (SOLVENT)

FIG. 9.

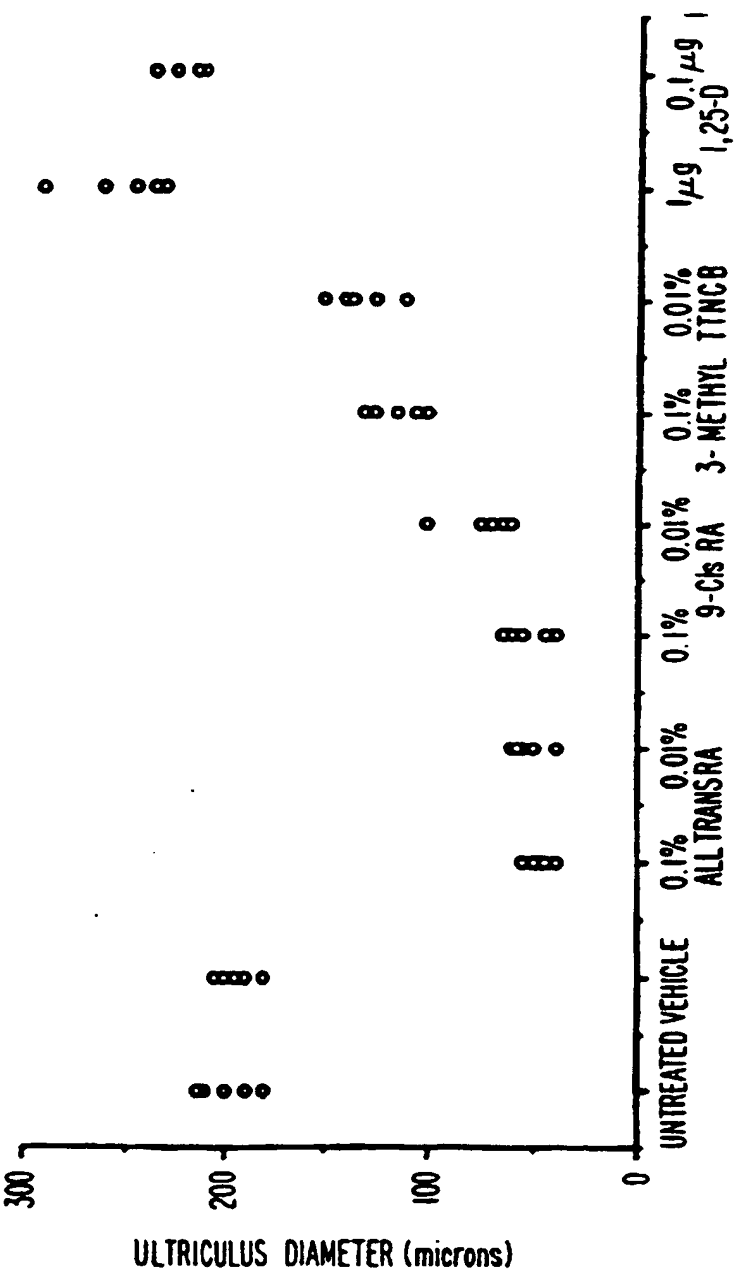

ULTRICULUS DIAMETER (microns)
300
200
100
0
UNTREATED
VEHICLE
0.1%
0.01%
ALL TRANS RA
0.1%
0.01%
9-Cis RA
0.1%
0.01%
3-METHYL TTNCB
1 μg
0.1 μg
1,25-D
TREATMENT GROUP

FIG. 10.

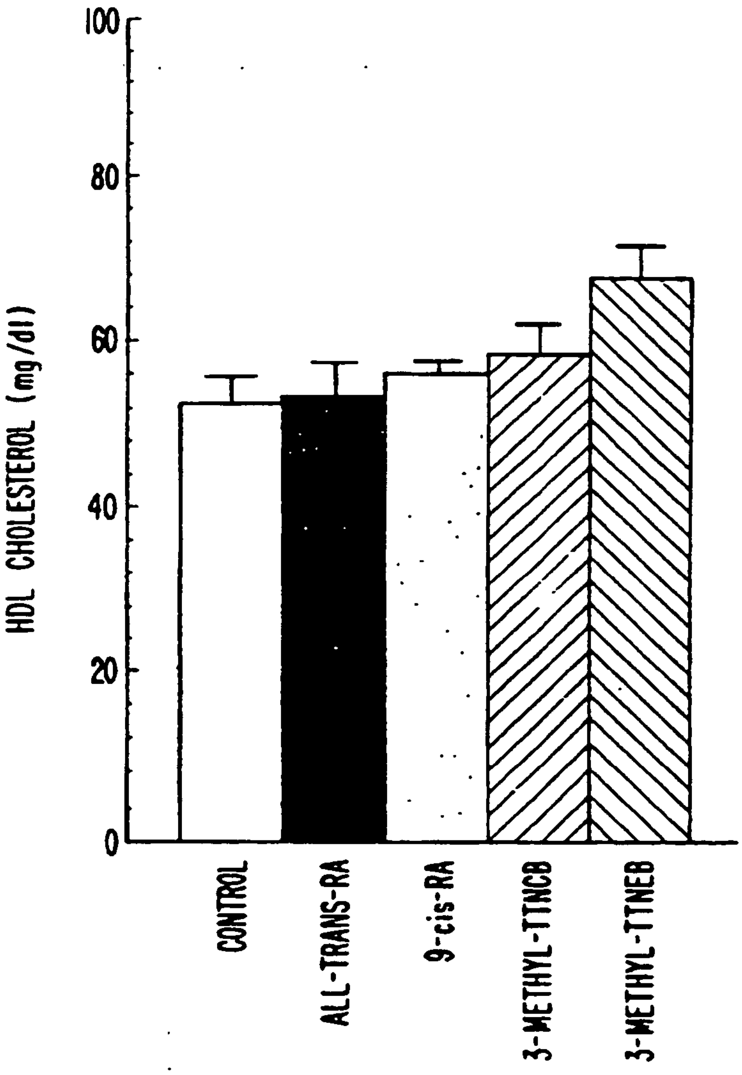
100
80
60
40
20
0
HDL CHOLESTEROL (mg/dl)
CONTROL
ALL-TRANS-RA
9-cis-RA
3-METHYL-TTNCB
3-METHYL-TTNEB

FIG. 11.

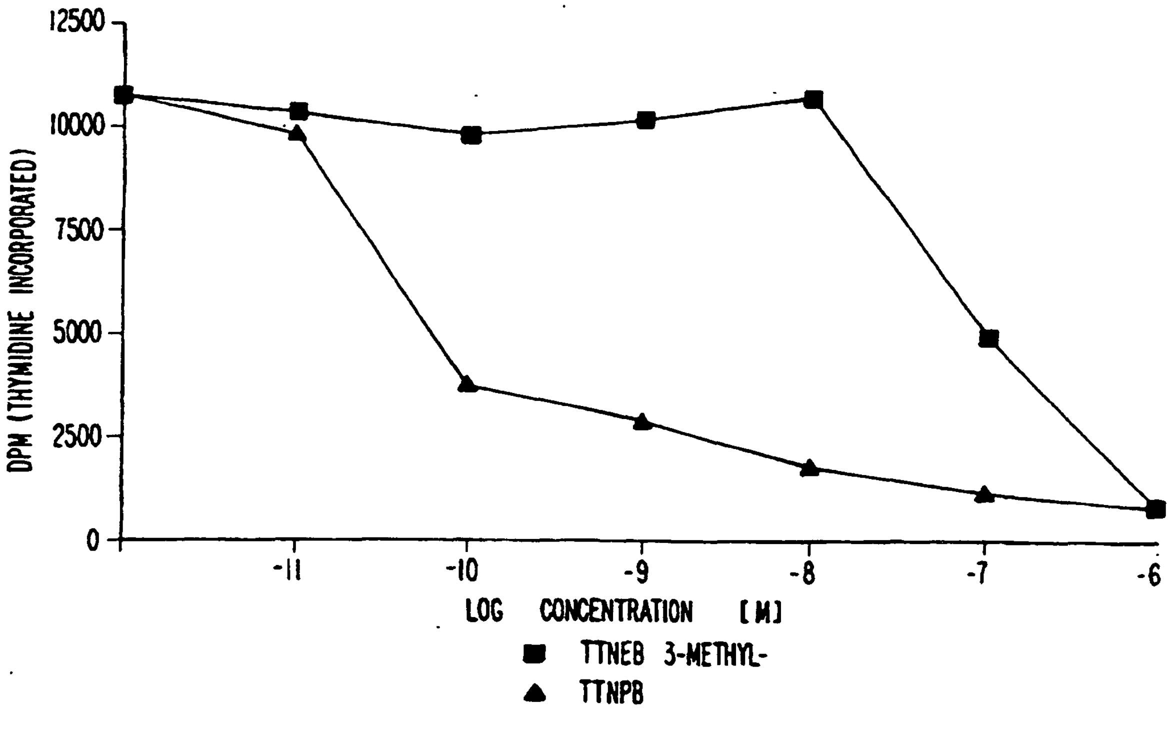
12500
10000
7500
5000
2500
0
DPM (THYMIDINE INCORPORATED)
-11
-10
-9
-8
-7
-6
LOG CONCENTRATION [M]
TTNEB 3-METHYL-
TTNPB

FIG. 12.

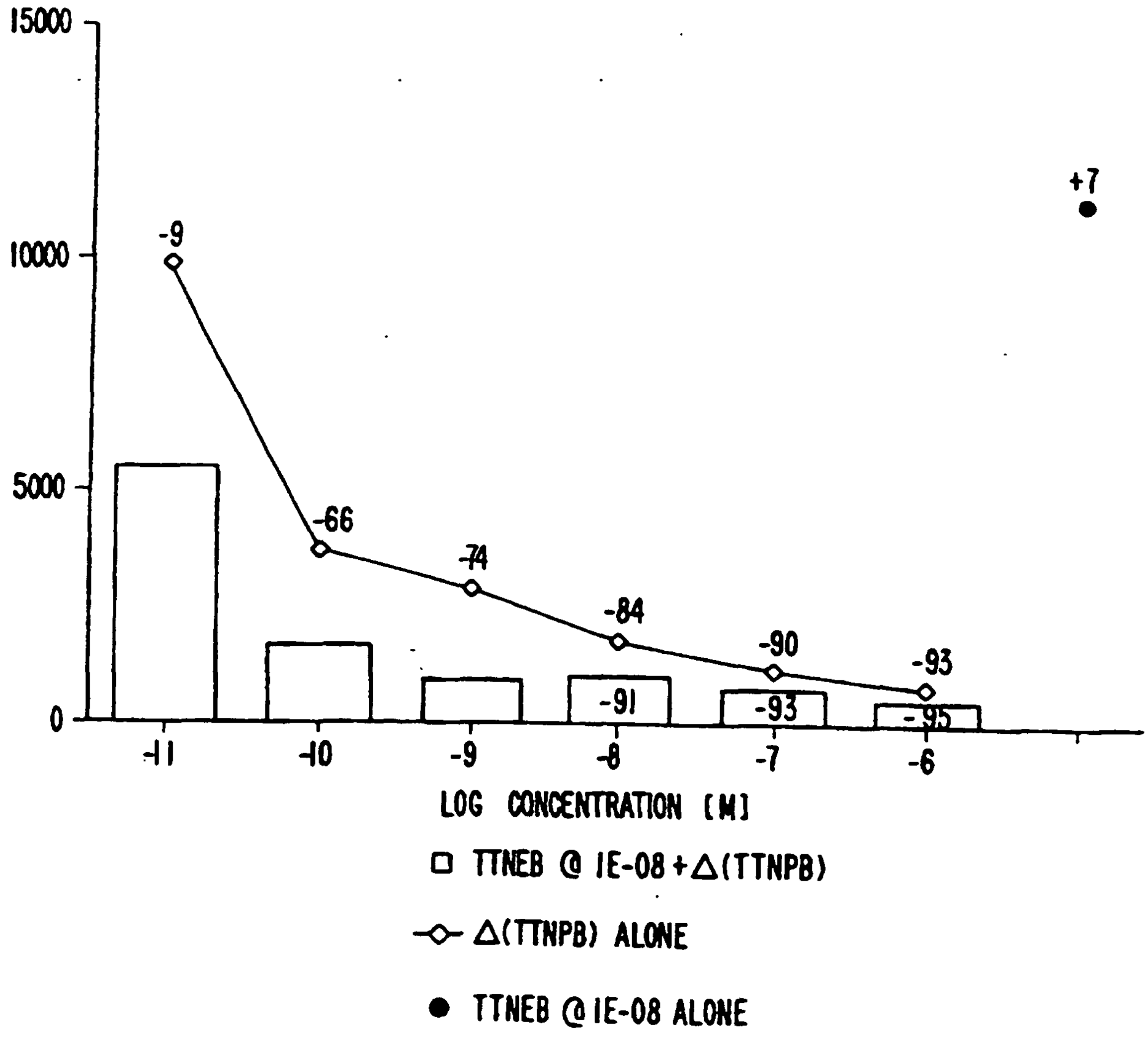
15000
10000
5000
0
-9
-66
-74
-84
-90
-93
+7
-91
-93
-95
-11
-10
-9
-8
-7
-6
LOG CONCENTRATION [M]
TTNEB @ 1E-08 + Δ(TTNPB)
Δ(TTNPB) ALONE
TTNEB @ 1E-08 ALONE